Europäisches Patentamt

European Patent Office

Office européen des brevets

(19)

(11) Publication number: **0 444 758 A1**

(12) **EUROPEAN PATENT APPLICATION**

(21) Application number: **91200422.3**

(22) Date of filing: **27.02.91**

(51) Int. Cl.⁵: **C12N 9/00, C12N 15/52,** C12N 1/21, C12N 1/15, C12P 35/00, C12P 37/00

The applicant has filed a statement in accordance with Rule 28 (4) EPC (issue of a sample only to an expert). Accession number(s) of the deposit(s): CBS 178.89, 179.89 and 142.90.

(30) Priority: **28.02.90 EP 90200488**
**28.02.90 EP 90200475**
**02.07.90 EP 90201768**
**03.10.90 EP 90202628**

(43) Date of publication of application:
**04.09.91 Bulletin 91/36**

(84) Designated Contracting States:
**DE FR GB NL**

(71) Applicant: **GIST-BROCADES N.V.**
**Wateringseweg 1 P.O. Box 1**
**NL-2600 MA Delft(NL)**

(72) Inventor: **Veenstra, Annemarie Eveline**
**Nierop 27**
**NL-2151 XH Nieuw Vennep(NL)**
Inventor: **Martin, Juan Francisco**
**Avda. de a Facultad No. 13-4 A**
**E-24004 Leon(ES)**
Inventor: **Garcia, Bruno Diez**
**C/Easo 25-4 D**
**San Sebastian (Guipuzcoa)(ES)**
Inventor: **Gutierrez, Santiago**
**C/Ildefonso F. Ordonez /24/1 -1Z9**
**Leon(ES)**
Inventor: **Barredo, Jose Luis**
**Trespaderne**
**Burgos(ES)**
Inventor: **Montenegro Prieto, Eduardo**
**C/Alvaro L pez N nez 12**
**E-24002 Leon(ES)**
Inventor: **Von Doehren, Hans**
**Schillerstrasse 34**
**W-1000 Berlin 12(DE)**
Inventor: **Palissa, Harriet**
**Chamissoplatz 3**
**W-1000 Berlin 61(DE)**
Inventor: **Van Liempt, Henk**
**Graefestrasse 55**
**W-1000 Berlin 61(DE)**

(74) Representative: **Matulewicz, Emil Rudolf**
**Antonius, Dr. et al**
**Gist-Brocades NV Patents and Trademarks**
**Department Wateringseweg 1 P.O. Box 1**
**NL-2600 MA Delft(NL)**

(54) **Method for modification of ACV synthetase and use of the modified enzyme for production of beta-lactam antibiotics.**

(57) Novel methods and compositions are provided for the enhanced production of fermentable or known and new secondary metabolites. The process is exemplified for penicillin. In addition, methods are provided for the production of modified P.chrysogenum δ-(L-α-aminoadipyl)-L-cysteinyl-D-valine Synthetase using recombinant DNA techniques.

EP 0 444 758 A1

## INTRODUCTION

### Technical Field

This invention relates to methods and compositions to provide and to enhance the in vivo and in vitro production of fermentable or known and new secondary metabolites, particularly β-lactams and their biosynthetic intermediates using recombinant DNA techniques.

### Background

β-Lactam antibiotics are the largest family of secondary metabolites produced in nature by microorganisms. The most important classes of the β-lactam antibiotics both clinically and economically are the penicillins (penam) and cephalosporins (cephem). Their biosynthesis occurs via a complex pathway of enzymatic steps; the unravelling of this pathway has been the subject of many studies during the last few decades. The first two steps are the key steps in the biosynthetic pathways of the penam and cephem classes of β-lactam antibiotics. After these two steps the biosynthetic pathways to the penicillins and cephalosporins diverge.

The first step in the biosynthesis of the penicillin, cephalosporin and cephamycin antibiotics is the condensation of the L-isomers of three amino acids, L-α-amino adipic acid (A), L-cysteine (C) and L-valine (V) into a tripeptide, δ-(L-α-aminoadipyl)-L-cysteinyl-D-valine or ACV. This step is catalyzed by the enzyme δ-(L-α-aminoadipyl)-L-cysteinyl-D-valine synthetase, hereinafter referred to as ACVS. In the second step, the tripeptide ACV is oxidatively cyclized by the action of the Isopenicillin N Synthase (hereinafter referred to as IPNS) or cyclase. The product of this reaction is Isopenicillin N; this compound contains the typical β-lactam and thiazolidine ring structures and possesses anti-bacterial activity. From Isopenicillin N the penicillins G or V are formed by exchange of the hydrophilic A side chain by a hydrophobic side chain. The side chains commonly used in industrial processes are either phenylacetic acid (PA), yielding penicillin G, or phenoxyacetic acid (POA), yielding penicillin V; this exchange reaction is catalyzed by the enzyme acyltransferase (hereinafter referred to as AT).

From Isopenicillin N the route to cephalosporin and the cephamycins proceeds via racemisation of the A side chain, forming penicillin N. This reaction is catalyzed by an enzyme named epimerase or racemase. The five-membered ring in penicillin N is expanded into a six-membered ring by the action of the enzyme deacetoxycephalosporin C synthase or expandase. The fungal enzyme has been shown also to catalyze the next reaction in the pathway, the hydroxylation of the methyl group at the 3'-position of the six-membered ring, forming deacetylcephalosporin C. In Streptomycetes this latter enzyme activity is encoded by a separate gene. From deacetylcephalosporin C, Cephalosporin C is formed by acetylation of the 3'-position. The cephamycins are formed from the same compound by several enzymatic steps.

In vivo synthesis of β-lactams and their precursors can be increased by increasing the activity of enzymes involved in β-lactam biosynthetic pathways. This can be achieved by either increasing the amount of enzyme present or by improving the specific activity of the enzyme. However, obtaining enzymes having the desired activity has typically been limited by the availability of spontaneous mutations in enzymes which are active at points in the pathway after either the rate limiting or the diverging steps. In addition, the biosynthesis of β-lactam antibiotics in general, and of penicillin in particular, is subject to glucose repression which hampers efficient production of these antibiotics and their precursors.

Currently, the cost effectiveness of in vitro synthesis of β-lactam precursors in cell-free extracts is poor in comparison to traditional fermentation processes. These in vitro processes are hampered by the limited amounts of enzyme present within the cell, and hence within the cell-free extract, and by the presence of inhibitory factors such as proteases and enzyme inhibitors. In addition, the use of fermentable or known β-lactam antibiotics (i.e. can be produced by fermentation of (β-lactam producing) non-recDNA microorganisms) is complicated by a) the development of resistance or tolerance to fermentable or known β-lactam antibiotics by bacterial species, and b) the limitations in therapeutic use, such as, an allergy to penicillin. There is, therefore, substantial interest in the development of systems which allow for the efficient and cost-effective production of both fermentable or known and new β-lactam antibiotics and their precursors, both in vivo and in vitro.

### Relevant Literature

Van Liempt et al. (J. Biol. Chem. (1989), 264:3680-3684) have shown in Aspergillus nidulans that the ACV condensation is carried out by a large multifunctional enzyme, ACV Synthetase. Similar results have been obtained for the Acremonium chrysogenum ACV Synthetase by Banko et al. (J. Amer. Chem. Soc. - (1987), 109:2858). The gene encoding ACV Synthetase (pcbAB) has been located on the genome of A.nidulans (MacCabe et al., EMBO J. (1990), 8:279-287) and Penicillium chrysogenum (EP-A-320272; EP-A-357119; D. Smith et al., Bio/Technology (1990), 8:39-41). pcbAB is located just upstream of the gene encoding IPNS.

In vitro synthesis of the tripeptide ACV in cell-free extracts (Adlington et al., Biochem. J. (1983), 213:573; G. Banko et al., J. Am. Chem. Soc. (1987), 109:2858; Jensen and Westlake, Developments in Industrial Microbiology (1989), 30:113-119; EP-A-280051) has been used to study parameters of the ACV Synthetase-reaction and also to study the feasibility of commercial application of in vitro synthesis of β-lactam antibiotics such as compared to traditional fermentation processes these processes are not commercially attractive (Jensen, supra). Several inhibitory compounds for the ACV Synthetase-reaction have been disclosed (Adlington and Banko, supra). It has also been established that the ACV Synthetase has a rather narrow substrate specificity. Only a few amino acids can be substituted for the native α-amino adipic acid, cysteine and valine.

Amplification of antibiotic biosynthetic genes by increased copy number resulting in an increase in the production of an antibiotic has been described by Skatrud et al., Bio/technology (1989), 7:477-485 and EP-A-357119, supra). Increased cephalosporin production, using the cefEF gene (Skatrud et al., supra) or penicillin, using the pcbC-penDE gene cluster (EP-A-357119) has been reported. Expression of β-lactam biosynthetic genes, other than the ACV Synthetase gene, has been described in Streptomyces lividans. (Chen et al., Bio/technology (1988), 6:1222-1224). Publications relating to enzymes included in β-lactam biosynthesis and the cloning of genes encoding these enzymes are as follows. The IPNS has been purified and the gene encoding this enzyme, pcbC, has been cloned from various organisms (reviewed in Miller and Ingolia, Molecular Microbiology 1989, 3:689-695; Martin and Liras, Advances in Biochemical Engineering/Biotechnology (1989), 39:153-187).

The enzyme acyltransferase has been purified (Alvarez et al., Antimicrob. Agents Chemother. (1987), 31:1675-1682; EP-A-336446) and the gene encoding this enzyme (penDE) has been cloned (EP-A-336446; EP-A-354624; Veenstra et al., in: C.L. Hershberger, S.W. Queener and G. Hegeman, eds: Genetics and Molecular Biology of Industrial Microorganisms, (1989), pp 262-269; Barredo et al., Gene (1989), 83:291-300). The pcbC and penDE genes are clustered in the genome of P.chrysogenum (B. Diez et al., Mol. Gen. Genet. (1989), 218:572-576; Veenstra, supra).

The epimerase which racemices the A side chain of isopenicillin N has been purified from Streptomyces clavuligerus (Usui and C-A Yu, Biochem. Biophys. Acta (1989), 999:78-85); the presence of this gene on a large DNA fragment was suggested in EP-A-233715. The enzyme expandase has been isolated both from A.chrysogenum (Kupta et al., FEMS Microbiol. Letters (1983), 16:1-6; Dotzlaf and Yeh, J. Bacteriol. (1986), 16:1611-1618) and from S.clavuligerus (Rollins et al., Can. J. Microbiol. (1988), 34:1196-1202) and Streptomyces lactamdurans (Cortes et al., J. Gen. Microbiol. (1987), 133:3165-3174). The expandase genes have been cloned from both A.chrysogenum (cefEF, Samson et al., Bio/technology - (1987), 5:1207-1214) and from S.clavuligerus (cefE, Kovacevic et al., J. Bacteriol. (1989), 171:754-760).

## SUMMARY OF THE INVENTION

Methods, and compositions for use therein, are provided for construction of genes encoding mutant ACVS, for enhanced expression of these mutant enzymes and for production of fermentable or known and new β-lactam antibiotics and precursors thereto. The methods include the steps of stably transforming a host cell with an expression cassette containing at least one DNA sequence encoding a biologically active mutant of ACV Synthetase (ACVS') and isolating transformants which produce the mutant ACV Synthetase. The expression cassette includes transcriptional and translational initiation and termination regulatory regions appropriate for the host cell. The host cell may be a eukaryote or a prokaryote. Also included are constructs and vectors comprising a mutant pcbAB gene, and transformed cells comprising the mutant pcbAB. The subject invention finds use particularly in improved production both in vivo and in vitro of fermentable or known and new β-lactam antibiotics and their precursors, particularly antibiotics of the penam and cephem classes.

## BRIEF DESCRIPTION OF THE FIGURES

Figure 1 shows a schematic representation of the distribution of β-lactam producing microorganisms.

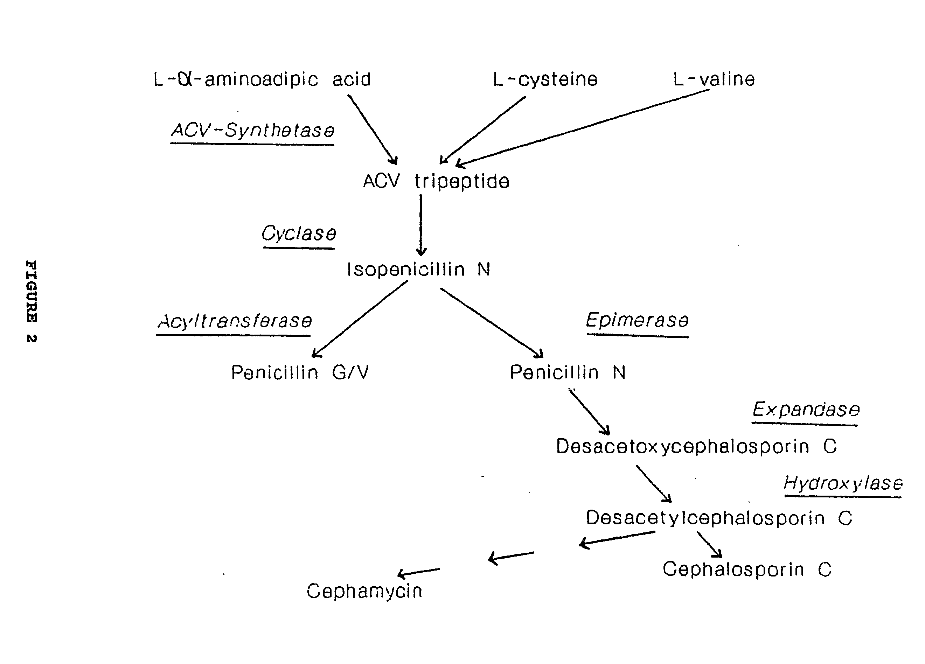

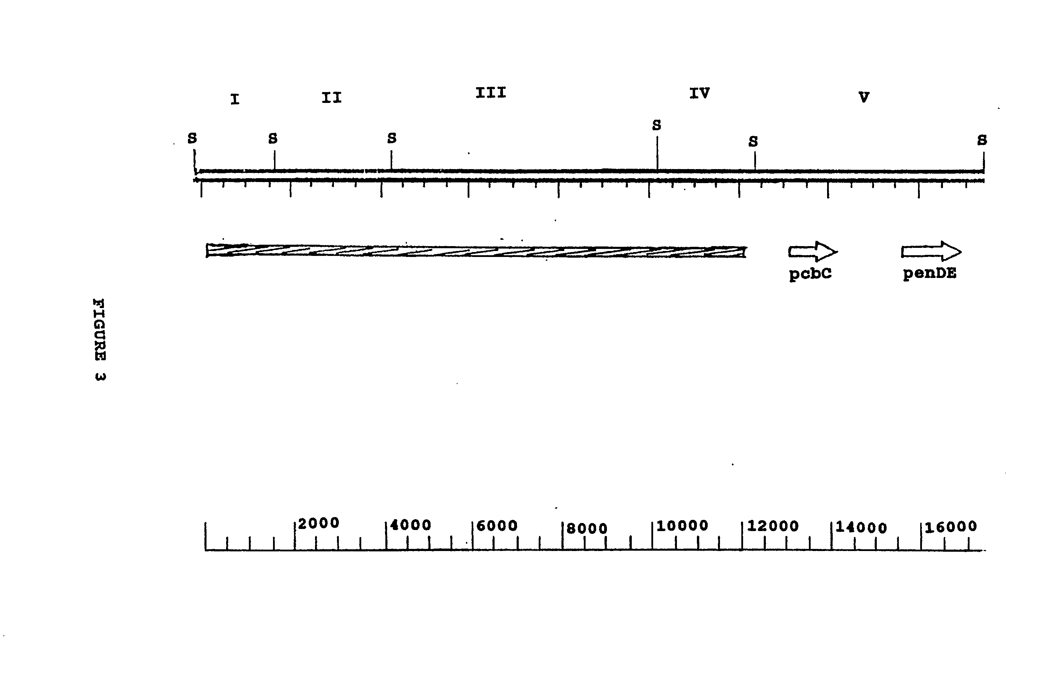

Figure 2 shows a schematic representation of the biosynthetic pathways of the penicillins, cephalosporins and cephamycins.

Figure 3 shows a restriction site and functional map of the chromosomal region containing the ACV Synthetase gene in P.chrysogenum. Probes that have been used for Northern hybridizations are indicated. The region containing the pcbAB gene is shaded. S = SalI.

Figure 4 shows a detailed physical map of the region encoding the ACV Synthetase gene in P.chrysogenum, including the sequencing strategy.

Figure 5 shows a schematic representation of the cluster of penicillin biosynthetic genes present in the genome of P.chrysogenum.

Figure 6 shows a restriction site and functional map of construct pPCV01.

Figure 7 shows a restriction site and functional map of construct pPCV02.

Figure 8 shows a restriction site and functional map of construct pPCV03.

Figure 9 shows a restriction site and functional map of the Escherichia coli vector pMAtrp.

Figure 10 shows a restriction site and functional map of pMA-ACV Synthetase.

Figure 11 shows a restriction site and functional map of pSLACV-01.

Figure 12A shows a restriction site and functional map of pSLACV-03A.

Figure 12B shows a restriction site and functional map of pSLACV-03B.

Figure 13 shows a scheme that shows the sequence homology between domains found within the ACVS protein and Gramicidin and Tyrocidin Synthetases.

Figure 14 shows a scheme that shows the sequence homology between the ACVS protein and the thioesterase domain of rat fatty acid synthetase.

Figure 15 shows a scheme listing the domains and sub-domains within the ACVS protein.


BRIEF DESCRIPTION OF THE SEQUENCE LISTING


Sequence listing 1 shows a nucleotide sequence and deduced amino acid sequence of the P.chrysogenum ACV Synthetase gene.


BRIEF DESCRIPTION OF THE SPECIFIC EMBODIMENTS


The first step in the biosynthetic pathway of the penam and cephem classes of $\beta$-lactam antibiotics is a key step in the formation of these antibiotics; the reaction is even considered to be rate determining (Van Liempt, supra). Until recently this reaction was thought to occur in two steps: formation of the dipeptide AC followed by addition of valine to form the tripeptide ACV (e.g. Nüsch, Heim and Treichler, Annu. Rev. Microbiol. (1987), 41:51-75). These steps were thought to be catalysed by two separate enzymes, encoded by the genes pcbA and pcbB. Results obtained by Van Liempt and Banko (supra), studying this reaction in A.nidulans and A.chrysogenum, respectively, indicate that tripeptide formation is catalysed by one multifunctional enzyme, tripeptide synthetase or ACVS. Multifunctional enzymes are defined herein to be enzymes that consist of one single polypeptide chain and that carry within their structure the ability to perform more than one catalytic reaction.

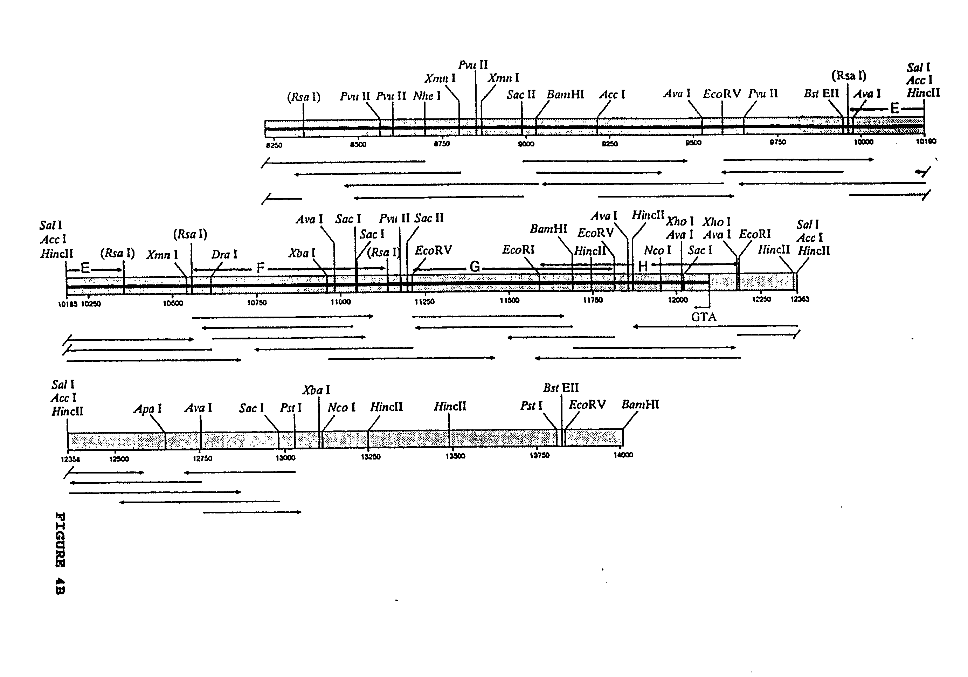

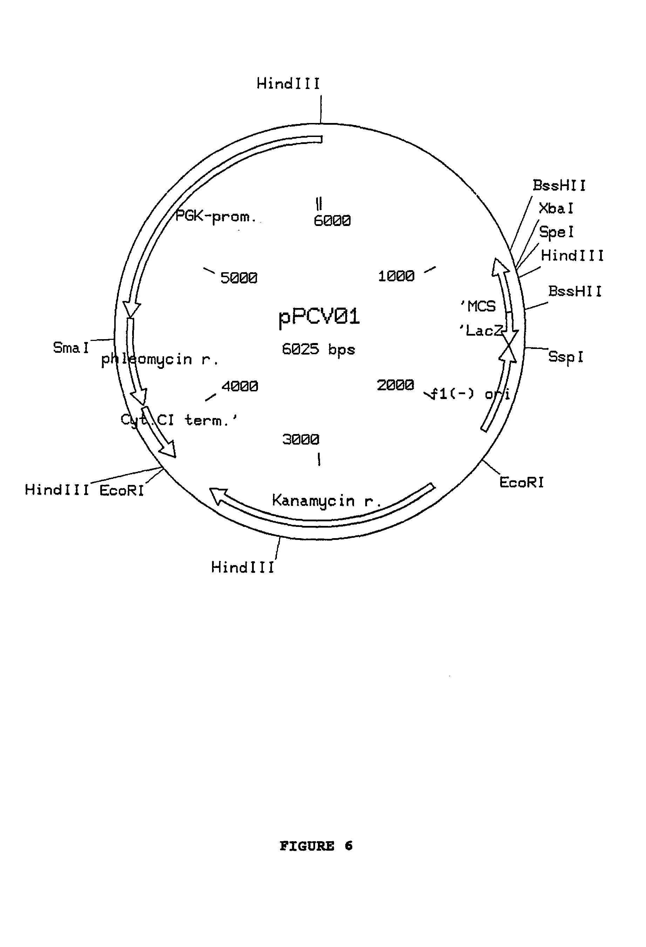

The existence of one enzyme indicates the presence of one gene encoding this enzyme, pcbAB. The localization of this gene in P.chrysogenum, just upstream of the gene encoding IPNS, is described in EP-A-357119 (non-prepublished). Similar results about the localization of the gene in P.chrysogenum are also described in EP-A-320272, and in Smith et al., supra. These data still allow for the ACVS activity to be encoded in more than one gene, however. Based on the results obtained for A.nidulans (250 kDa enzyme: Van Liempt et al.) and P.chrysogenum (gene present on 8 x 10³ nucleotides DNA fragment: EP-A-320272) a gene with a size of at most 8 x 10³ nucleotides would be predicted. Surprisingly, the data presented in the present patent application indicate that the coding region of the P.chrysogenum pcbAB gene is 11,337 nucleotides long and encodes a protein of 413 kDa. In Figure 6B of EP-A-320272 a restriction map of the DNA comprising the asserted intact ACVS gene has been given. From the expression of the 8 kb EcoRI fragment in the vector pPEN3 it is concluded that this fragment contains one or more genes that are involved in the ACV Synthetase production. Comparison of the restriction map (Figure 6B of EP-A-320272) with the one in this patent application (Figure 4) clearly demonstrates that the EcoRI fragment of EP-A-320272 encodes an incomplete ACV Synthetase lacking the first about 500 nucleotides (about 170 N-terminal amino acids) and the last about 2900 nucleotides (about 970 C-terminal amino acids). This is not recognized by the applicants of EP-A-320272. Besides that, a significant amount of ACV Synthetase can now be achieved by using the manipulated pcbAB genes of the present invention.

Only now it can be envisaged that all catalytic activities required for tripeptide formation (i.e. activation of three amino acids, racemisation of valine, formation of dipeptide AC and tripeptide ACV, release of ACV) can indeed be present in one single polypeptide chain. ACVS hence is a multifunctional enzyme.

Multifunctional enzymes are defined herein to be enzymes that consist of one single polypeptide chain and that carry within their structure the ability to perform more than one catalytic reaction. In contrast, in the case of the biosynthesis of Gramicidin S, a peptide antibiotic consisting of two identical units of five amino acids, at least two, and probably three polypeptide chains are involved (e.g. Von Döhren, 1982, In: Peptide Antibiotics, W. de Gruyter & Co., Berlin. pp 169-182; Krätzschmar et al., Journ. of Bacteriol. (1989), 171, 5422-5429). Tyrocidine synthetase involved in the biosynthesis of the decapeptide tyrocidine also contains three subunits (Mittenhuber, et al., J. Bacteriol. (1989), 171:4881-4887). Several genes encoding subunits of both enzymes, have been cloned and sequenced (Krätzschmar, supra; Mittenhuber, supra).

In accordance with the subject invention, methods and compositions are provided which allow for expression of biologically active mutants of ACV Synthetase and for increased, cost-effective production of fermentable or known and new β-lactam antibiotics and their precursors both in vivo and in vitro. The method includes the steps of transforming a host cell using an expression cassette which includes in the 5'-3' direction of transcription, a transcriptional regulatory region and translational initiation region, an open reading frame encoding a mutant ACV Synthetase (ACVS'), optionally having a signal sequence for secretion recognized by the host cell; and translational and transcriptional termination regions. The initiation and termination regulatory regions are functional in the host cell and provide for efficient expression of ACVS' without undesirable effects on the viability and proliferation of the host cell. Transcription and translation of the ACVS' DNA sequence will provide for expression of mutant ACV Synthetase in the host cell, which may have novel or enhanced properties as compared to the native enzyme.

Optionally, the expression cassette may include a transcription regulating sequence which is not subject to repression by substances present in the growth medium. The expression systems may be used to prepare existing or novel β-lactam antibiotics directly or they may be used to prepare cell-free extracts containing large quantities of ACVS' for in vitro preparation of the antibiotics. Alternatively, the expression hosts may be used as a source of large quantitites of ACVS' which can be purified and used in in vitro systems.

In currently used systems, the first step in the in vitro synthesis of new (i.e. those which do not occur in nature) -lactams or their precursors, has been the chemical synthesis of the desired new tripeptide; these modified tripeptides are subsequently cyclised by the action of the IPNS enzyme. The availability of ACVS' offers the advantage that the laborious chemical synthesis of new tripeptides can be avoided; the substantially more efficient and cost effective enzymatic synthesis of the desired tripeptides can be used instead. Additionally, by avoiding the use of harmful chemicals, solvents and the like, the subject invention causes less environmental problems when carried out at an industrial scale than do traditional methods of synthesis.

Recognition of some of the altered tripeptides as a substrate by IPNS, however, may in turn require the design and use of a modified IPNS enzyme.

Production of ACVS' is achieved by the introduction of at least one copy of a mutant gene encoding ACV Synthetase (pcbAB' gene) into a host cell by transformation: protoplasts are mixed with DNA constructs that contain at least one copy of the mutant gene linked to a selectable marker. By choosing the appropriate conditions, some protoplasts will take up the DNA construct which is thereafter stably maintained because the construct has become integrated into the host cell genome. Transformed cells can be selected from the background of so-called "non-transformed" cells by screening for expression of the selectable marker. Expression of the pcbAB' gene, which may be used to produce modified cephalosporin, penicillin, and cephamycin thus can be expected to result in intracellular enzyme activity, which in turn will be accompanied by production of the desired modified antibiotics. Since ACV Synthetase forms part of the biosynthetic pathway of both the penam and the cephem classes of β-lactam antibiotics, production of ACVS' has several applications.

For preparation of ACVS' and modified secondary metabolites, or for preparation of ACVS' by recombinant methods, genes encoding ACVS' may be obtained from spontaneous pcbAB' mutants from a variety of sources including Penicillium chrysogenum, Acremonium chrysogenum, Aspergillus nidulans, or from Flavobacterium or the Streptomycetes. Also, sequences that encode biologically active mutant ACV Synthetase can be derived from sequences that are at least substantially identical to the sequence of a fermentable or known ACV synthetase or from spontaneous mutant pcbAB alleles. Mutant sequences can be "derived" by a variety of genetic and recombinant DNA techniques, such as in vitro mutagenesis and homologous recombination. By "substantially identical" is intended sequences which can include conservative mutations, where the sequence encodes the same amino acid sequence, but may have as many as 30% different nucleic acid bases, more usually not more than 10% different bases, or mutations which are non-conservative, where fewer than about 10%, more usually fewer than about 5%, and preferably not more than 1% of the encoded amino acids are substituted or deleted; and there are fewer than 5% of inserted amino acids, where the percentage is based on the number of naturally occurring amino acids in the native enzyme.

The gene of interest encodes biologically active mutant ACV Synthetase or a biologically active portion of ACV Synthetase or a mutant thereof. A spontaneous mutant gene or a wild-type gene (from which a mutant structural gene subsequently can be derived) can be isolated by various techniques using host organisms which are ACV Synthetase mutants or wild type. The techniques can include isolating mRNA from a host organism which codes for the polypeptide of interest, the mRNA reverse transcribed, the resulting single stranded (ss) DNA used as a template to prepare double stranded (ds) DNA and the dsDNA gene isolated.

Another technique is to isolate a piece of the host cell DNA and, using a probe, appropriately degenerate, comprising a region of the most conserved sequences in the gene of interest, identify sequences encoding ACV Synthetase in the host cell genome. The probe can be considerably shorter than the entire sequence, but should be at least 10, preferably at least 14, more preferably at least 20 nucleotides in length. Longer nucleotides of the gene of interest are also useful. Both DNA and RNA probes can be used.

In use, the probe is typically labeled in a detectable manner (for example with $^{32}$P or biotinylated nucleotides) and are incubated with ss DNA or RNA from the organism in which the gene is being sought after separation and/or immobilization of the ss or ds DNA, typically using nitrogencellulose paper or nylon membranes. Hybridization is detected by means of autoradiography. Hybridization techniques suitable for use with oligonucleotides are well known to those skilled in the art. Although probes are normally used with a detectable label that allows for easy identification, unlabeled oligonucleotides are also useful, both as precursors of labeled probes and for use in methods that provide for direct detection of DNA or DNA/RNA. Accordingly, the term "oligonucleotide" refers to both labeled and unlabeled forms.

Genes encoding ACVS or ACVS' can also be isolated from other organisms and modified as appropriate. For the gene encoding the IPNS, it has been shown (See for example, Ingolia and Queener, Medicinal Research Reviews (1989) 9:245-264) that genes isolated from different organisms show a high degree of homology, ranging from about 70% on the DNA level if two fungal or two Streptomyces genes are compared to 60% or more if a fungal and a Streptomycete gene are compared. Homologies on the protein level are 75% and 54%, respectively. Despite the differences on the DNA and protein level, all IPNS proteins catalyse the same reaction in a similar fashion. Therefore, ACVS and ACVS' sequences and enzymes can be identified that share a minimal homology of about 60% on the DNA level or 50% on the protein level.

ACVS and ACVS' synthetase genes can be isolated by using, for example, the sequence as given in Sequence listing 1, or parts thereof as a probe in heterologous hybridization experiments. These probes can be restriction fragments derived from the ACVS encoding DNA isolated from P.chrysogenum; such restriction fragments can easily be selected and isolated using the restriction map as given in Figure 4. Alternatively, synthetic oligonucleotide probes can be made based on the data given in Sequence listing 1. In still another variation, oligonucleotides can be designed, based on the data in Sequence listing 1, and used in a PCR reaction to generate a larger probe fragment. In this way, for example, the genes of A.chrysogenum and A.nidulans can be readily isolated; also the genes from Streptomycetes can be isolated using less stringent hybridization conditions. The genes that are isolated in this fashion are also of interest.

Mutations in ACV Synthetase coding regions are also obtained directly from organisms (from which at least one allele of ACV Synthetase has been previously isolated as described above) by homologous recombination techniques or PCR (polymerase chain reaction) technology (see for example various authors in: PCR Technology, Principles and Applications for DNA Amplification, 1989. H.A. Erlich (ed). Stockton Press). Thus, genetic selection methods can be devised which yield ACV Synthetase mutants with desirable properties, and subsequently the mutated region is quickly located and retrieved without resorting to cloning and sequencing of the entire mutant allele.

Alternatively, the DNA sequences encoding the mutant ACV Synthetase can be synthesized using conventional techniques such as PCR (Polymerase Chain Reactions) or by synthesis of overlapping single strands which may be ligated together to define the desired coding sequences. The termini can be designed to provide restriction sites or one or both termini may be blunt-ended for ligation to complementary ends of an expression vector. For expression of the sequence an initial methionine is provided. Expression vectors are generally available and are amply described in the literature.

Once the ACV Synthetase DNA is obtained, mutations can be introduced by a number of in vitro mutagenesis techniques, either random or site-directed. Precise changes to the amino acid sequence are obtained by site-directed mutagenesis techniques which use synthetic oligonucleotides complementary to the region to be modified, except for the desired nucleotide(s) change. Regions are precisely deleted by "loop-out" mutagenesis techniques using synthetic oligonucleotides. Precise insertions are obtained using synthetic oligonucleotides to generate appropriate restriction sites. A series of mutations localized to a region of the ACV Synthetase-encoding DNA sequence on a plasmid are generated by in vitro mutagenesis techniques and subsequently identified by cloning and sequencing the isolated mutagenized plasmids.

Once specific mutations have been generated and isolated to individual plasmids, "cassette" mutagenesis is applied to generate a series of new mutants by combining mutations into the same plasmid using appropriate restriction sites. Alternatively, mutagenesis using two or more oligonucleotides directed to different regions of the gene yields mutants with the desired multiple mutations.

Mutant proteins are also generated by the insertion, addition, or substitution of coding sequences from other proteins to ACV Synthetase coding sequences. The source proteins may be ACV Synthetases from other strains or species or may be unrelated proteins. Such amino acid sequences introduced into ACV Synthetase will impart desirable properties or characteristics which originally belonged to the source protein.

Desirable properties are useful properties for activities and functions which include and are not limited to protein stability, secretion, isolation, purification, increased enzymatic activity, resistance to inhibitors, resistance to heat inactivation, proteases and denaturants, solubility, and modified substrate specificity. Source proteins include those proteins involved in amino acid activation, amino acid racemization, peptide formation, thioesterase activity and the like. Preferred proteins include proteins involved in the biosynthesis of other antibiotic peptides, such as tyrocidin synthetase, gramicidin synthetase or proteins having enzymatic activities similar to those of ACVS, such as long chain fatty acid synthetases.

Although any region of the protein may be mutated, regions of the ACV Synthetase protein which serve as candidates for mutagenesis are defined in order to minimize extensive screening of randomly generated mutants. The nucleotide and deduced amino acid sequences of ACV Synthetase provided in the application are a powerful tool with which to delineate such regions. Sequence homology comparisons, at both the DNA and amino acid level, identify protein regions with known function or structure.

Hydrophobicity profiles or related biophysical profiles, based on the amino acid sequence, with subsequent comparisons to profiles of other proteins may be used to identify additional protein regions of known structure and function. The finding of sequence or profile homologies indicates that the protein regions share a similar function, activity or enzymatic mechanism. Hydrophobicity or secondary structural profiles can indicate domain "linking" regions, "hinge" regions or "loops" which are candidates for restriction site insertions to generate domain "cassettes".

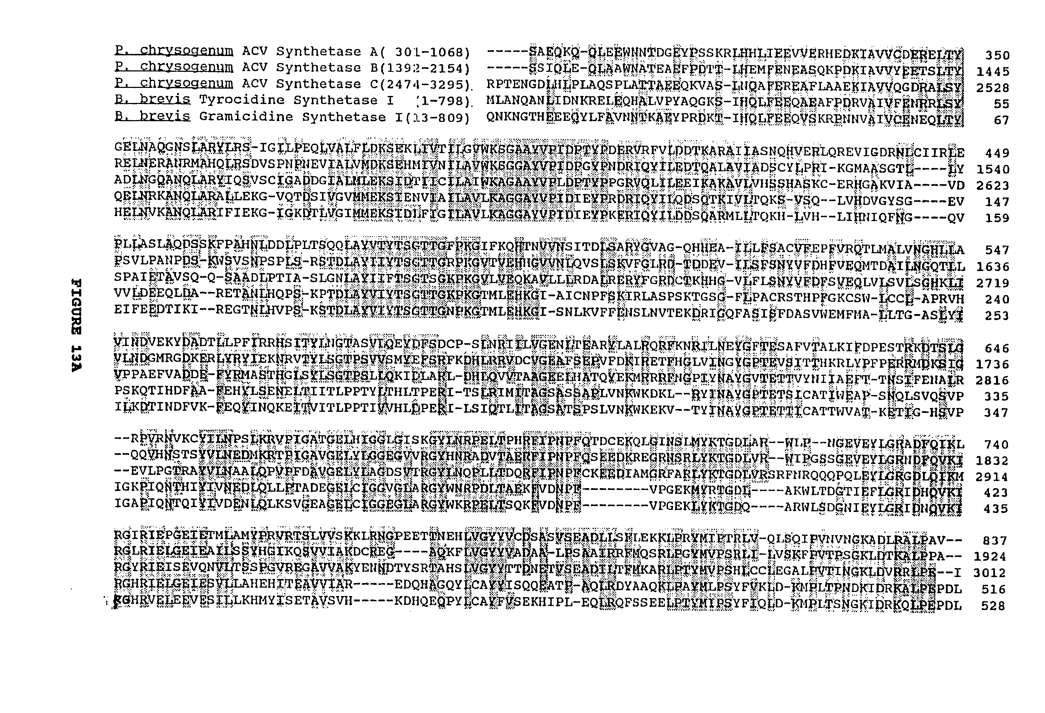

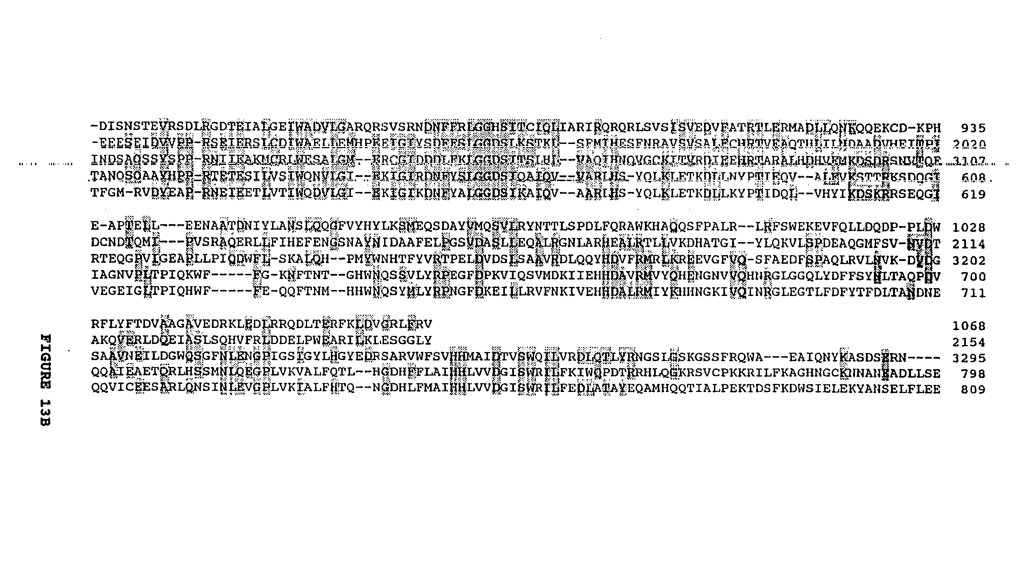

Regions for mutagenesis or replacement are also defined by correlation of a genetic map of ACV Synthetase mutants alleles with a physical map, either at the restriction site or sequence level. Preferred regions for mutagenesis, either site-directed or via substitution include the functional domains and sub-domains of ACV Synthetase as provided in Figures 13, 14, and 15.

Mutant proteins also include functional proteins formed by various combinations or quarternery assemblies of discrete polypeptides. The polypeptides are expressed individually, either from the same or different plasmids, in a host cell or isolated and recombined in vitro. The polypeptides contain one or more enzymatic activities. Preferably the polypeptides comprise one or more of the domains of ACV Synthetase as defined in Figures 13, 14, and 15. Polypeptides from corresponding regions of other proteins, as defined in the preceding sections, may also be combined with one or more ACV Synthetase polypeptides in the same discrete fashion.

Once the desired mutant DNA sequence has been obtained, it may be manipulated in a variety of ways to provide for expression. It is highly desirable that modifications of the nucleotide sequence, other than the modifications which result in the desired mutation(s), retain the three dimensional structure of the expression product, particularly that portion of the structure which may be responsible for the enzymatic activity of the resulting enzyme. For example, convenient restriction sites may be designed into the DNA sequence of interest; when possible the restriction site(s) leaves the amino acid sequence of the expression product unaltered.

Where the mutant gene encoding ACV Synthetase is to be expressed in a host which recognizes the wild-type transcriptional and translational regulatory regions of the gene of interest, the entire gene with its wild-type 5' and 3'-regulatory regions may be introduced into an appropriate expression vector. Where the mutant gene is to be expressed in a host cell which does not recognize the naturally occurring wild-type transcriptional and translational regulatory regions, further manipulation may be required. Conveniently, a variety of 3'-transcriptional regulatory regions are known and may be inserted downstream from the stop codons. The non-coding 5'-region upstream from a structural gene may be removed by endonuclease restriction, Bal31 restriction or the like. Alternatively, where a convenient restriction site is present near the 5'-terminus of the structural gene, the structural gene may be restricted and an adaptor employed for linking the structural gene to the promoter region, where the adapter provides for the lost nucleotides of the structural gene. Thus, the initiation and termination regions may be homologous (derived from the original host), or heterologous (derived from a foreign source or synthetic DNA sequences). The expression cassette therefore may be wholly or partially derived from natural sources, and either wholly or partially derived from sources homologous to the host cell, or heterologous to the host cell. The various DNA constructs (DNA sequences, vectors, plasmids, expression cassettes) of the invention are isolated and/or purified, or synthesized and thus are not "naturally occurring". By foreign is intended that the transcriptional initiation region is other than the transcriptional initiation region associated with a structural gene for ACV. The transcription regulatory region may include an element that stimulates transcription, known in the art as "enhancer", or may contain an element that can be regulated by the addition or omission of certain elements from the growth medium.

Illustrative transcriptional regulatory regions or promoters which find use in the subject invention, include, for prokaryotic cells, the lac, trp (Sommerville, Biotechnology and Genetic Engineering Reviews - (1988), 6:1-41) or tac promoters of E.coli, or aph or tyrosine synthetase promoters of S.lividans. For filamentous fungi, illustrative promoters include the glyceraldehyde phosphate dehydrogenase (gapdh) promoter, the phosphoglycerate kinase (pgk) promoter, the nitrate reductase promoter and the like. A preferred embodiment of the present invention which is exemplified herein is the use of the pgk promoter of P.chrysogenum, which has been described in EP-A-354624.

The biosynthesis of β-lactam antibiotics in general, and of penicillin in particular, is subject to glucose repression (Martin and Liras, TIBS (1985), 3:39-44). This repression by glucose has been unequivocally established for the formation of the tripeptide by the ACV Synthetase and for the activity of IPNS (Revilla et al., J. Bacteriol. (1986), 168:947-952). It is not known at which stage of expression repression by glucose is exerted; this can, for example, be at the transcriptional or at the translational level. If the former applies, constitutive expression of the pcbAB gene will result in an increase in enzyme activity followed by an increase in the production of ACV, and subsequently of the β-lactam antibiotic derived from it.

Increased expression of the gene in β-lactam producing bacterial or fungal hosts therefore can be obtained by changing the regulation of gene expression. Thus, the transcriptional regulatory region is preferably one which is not subject to repression by, for example, presence or absence of nutrients such as glucose, or expression products in the growth medium. The transcriptional regulatory region may additionally include regulatory sequences which terminate transcription and which provide sequences or structures which inhibit degradation of the mRNA.

Exemplary of changing the regulation of expression is modification of the pcbAB gene. The native sequence generally is replaced by a region which is functional in either the native or heterologous host and wherein expression is either inducible or constitutive. For example, the regulatory sequences can be changed by replacing the pcbAB promoter, which is strongly repressed by glucose, with a promoter which is insensitive to glucose or even is stimulated by it. In the latter situation, antibiotic will be produced during the early stages of the fermentation, when biomass is formed in high-glucose conditions. This modification may further increase the yield of the antibiotic during the fermentation. Expression of the gene may also be brought under control of other promoters, either promoters for which expression can be regulated in a different fashion or promoters that are expressed constitutively.

In eukaryotic cells, transcriptional termination regions provide for proper maturation of the mRNA transcript and are necessary for efficient expression. In general, it is preferable to use the native polyadenylation signal associated with the gene of interest. In both eukaryotic and prokaryotic systems, termination regions can also contain sequences or structures which increase the stability of the mRNA species and allow for higher expression. Several examples of prokaryotic sequences are known, for example the trp terminator, the gene 32 (T4) terminator, or synthetic terminators which are similar in sequence to gene 32. For eukaryotes, terminators can be used that are isolated from cloned genes. For yeast, the terminator of the CYC1 gene or the actin gene can be used for example. For filamentous fungi, the terminators isolated from for example the trpC gene, the pgk gene or the penDE gene are useful.

Where it is desired to isolate the ACVS, secretion of the enzyme into the media or into the periplasmic space of the transformed microbial host can improve the efficiency of the isolation procedure. Secretion can be accomplished by using DNA expression cassettes as described herein, which further comprise a signal sequence (secretory leader) that is functional in the host cell. The signal sequence will be heterologous to the ACV Synthetase gene and may be homologous or heterologous to the host cell, or may be a synthetic signal sequence. The signal sequence provides a peptide sequence that is in-frame with the enzyme sequence, and may be located 5' or 3' to the ACV Synthetase sequence. The signal sequence can also be provided by joining, in-frame, an open reading frame of a protein that is secreted by the host cell and the open reading frame of the ACV Synthetase. Illustrative secretory leaders include the secretory leaders of

penicillinase, α-factor, immunoglobulin, T-cell receptors, outer membrane proteins, glucoamylase, fungal amylase and the like. By fusion in proper reading frame, the mature polypeptide may be secreted into the medium.

The initiation and termination regions may be homologous (derived from the original host), or heterologous (derived from a foreign source or synthetic DNA sequences). The expression cassette thus may be wholly or partially derived from natural sources, and either wholly or partially derived from sources homologous to the host cell, or heterologous to the host cell. The various DNA constructs (DNA sequences, vectors, plasmids, expression cassettes) of the invention are isolated and/or purified, or synthesized and thus are not "naturally occurring."

The expression cassette may be included within a replication system for episomal maintenance in an appropriate cellular host or may be provided without a replication system, where it may become integrated into the host genome. Integration may be stimulated in yeast and bacteria by the inclusion of ribosomal RNA genes or other yeast genes and subsequent linearization in these genes.

The DNA may be introduced into the host cell in accordance with known techniques, such as transformation DNA, transfection by contacting the cells with a virus, micro-injection of the DNA into cells and the like. Both prokaryotic and eukaryotic hosts may be employed, which may include bacteria and fungi, particularly filamentous fungi. Prokaryotic cells include Escherichia coli, Flavobacterium and Streptomyces spp. Eukaryotic cells include filamentous fungi such as Penicillium chrysogenum, Acremonium chrysogenum, Aspergillus nidulans, niger and oryzae; and yeasts such as Saccharomyces cerevisiae, Kluyveromyces lactis. Preferred host cells include P.chrysogenum, A.chrysogenum, A.nidulans since they contain the entire biosynthetic pathway. Replacement of the native ACVS gene by the mutant gene may result in in vivo synthesis of β-lactam derivatives. The presence of a wild-type gene in these organisms indicates that they allow for efficient expression of the wild-type ACVS gene and hence they are inferred to also express the mutant gene without too many difficulties.

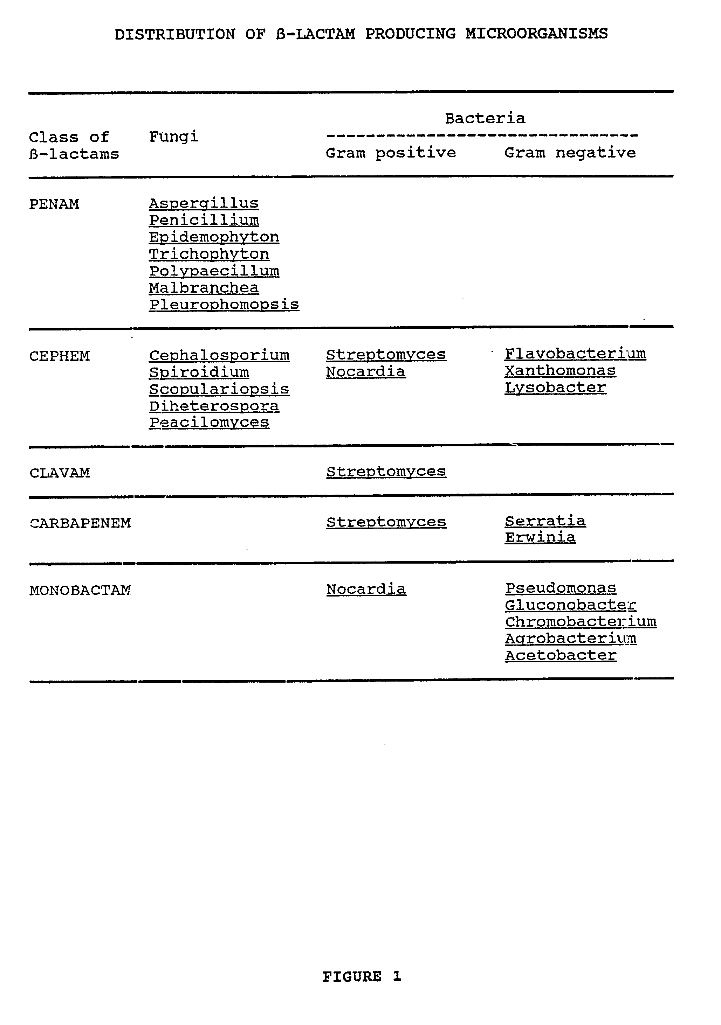

Other preferred hosts include the Streptomycetes. Several Streptomycetes also synthesize β-lactams (see Figure 1). They can be used for the same reasons as the above-mentioned fungi. On the other hand, a Streptomycete such as S.lividans is very amenable to genetic manipulation (D.A. Hopwood et al. (1985), Genetic Manipulation of Streptomyces: a Laboratory Manual. The John Innes Foundation, Norwich, U.K.). Moreover, it has been disclosed that β-lactam biosynthetic genes are actively expressed in S.lividans (Chen et al., supra). Therefore, S.lividans is a preferred host for expression of the gene, for isolation of the mutant protein, or to use for preparation of cell-free extracts. Expression of foreign genes in E.coli is very well known in the art. One disadvantage of this organism may be that the ACVS could be too large for production of active enzyme; inclusion bodies may be formed or (over)expression may be harmful to the cell. Yeasts, like S.cerevisiae or K.lactis are examples of frequently used hosts for expression of heterologous proteins.

For synthesis of fermentable or known and new β-lactam antibiotics and intermediates in the biosynthesis of such β-lactams, such as the tripeptide δ-(L-α-aminoadipyl)-L-cysteinyl-D-valine, or new tripeptides, such as D-phenyl-alanyl-L-cysteinyl-D-valine, various methods may be used. The mutant ACV Synthetase enzyme can be isolated from the cultures of the transformed host cells as described above and used in in vitro systems to synthesize the secondary metabolites. Alternatively, the transformed host cells can be used directly to produce the desired secondary metabolite. Transformants of filamentous fungal hosts are grown under conditions that are suitable for the antibiotic production. These conditions have been amply described in the literature (Luengo et al., J. Gen. Microbiol. (1979), 115:207-211; Barredo et al., Antimicrob. Agents Chemother. (1988), 32: 1061-1067; Queener and Schwartz, In: Rose AH (ed) Secondary Products of Metabolism, Academic Press, London (1979): 35-122; Queener et al., In: Biotechnology of Industrial Antibiotics, EJ Vandamme (ed) Marcel Dekker Inc., New York, Basel (1984): 141-170). Generally, media for antibiotic production contain either a slow fermentable carbon source, like lactose, or are limited in the carbon source, e.g. glucose, in a so called fed-batch fermentation procedure. For the production of penicillin G or V the appropriate side chain precursor, as has been described hereinbefore, is added to the medium; for the production of cephalosporin the medium may be supplemented with DL methionin. Generally, Corn Steep Liquor or related compounds are supplied as a nitrogen source.

For prokaryotic hosts, the culture conditions are known in the art (T. Maniatis et al., Molecular Cloning, a Laboratory Manual, Cold Spring Harbor Laboratory (First edition, 1982 or second edition, 1989; and Hopwood et al. (supra)); in this case it is advisable to maintain selective pressure by addition to the culture medium of the antibiotic that is used as a selective agent. The secondary metabolite is subsequently isolated from the cultured cells.

ACVS' can be purified from the host cells that have been grown under these conditions or, alternatively, using the conditions that are most suited for the expression of the promoter used; preferably, cells are harvested early in the fermentation (2-4 days) for this purpose. For the isolation of ACVS', the procedures as described in Van Liempt et al. (supra) or Banko et al. (supra) can be used. For isolation of ACVS' from transformed E.coli, the cells are grown overnight in e.g. TY or LB medium (Maniatis et al., supra) and can be lysed by treatment with lysozyme. Protease inhibitors, such as PMSF or α-2 macroglobulin can be included in the buffers used, in order to avoid degradation of the ACVS' to be purified.

Cell-free extracts can be prepared from the various cultures using the procedures as they are described in Adlington et al. (supra), Banko et al. (supra), Jensen et al. (supra), Zhang and Demain (Biochem. Biophys. Res. Comm. (1990) 196: 1145-1152), Jhang et al. (FEMS Microbiol. Lett. (1989) 57: 145-150). In general, cells are harvested, washed in a suitable buffer and disrupted in a French press, by grinding in liquid nitrogen or by sonication. The presence of glycerol (40-50%) as a stabilizer is crucial, both for isolation of large quantities of the enzyme and for isolation of an active cell-free extract.

It has been shown that amplification of antibiotic biosynthetic genes can result in an increase in the production of an antibiotic. Increased cephalosporin production, using the cefEF gene (Skatrud et al., Bio/technology (1989), 7:477-485) or penicillin, using the pcbC-penDE gene cluster (EP-A-357119 (non-prepublished)) has been reported. Amplification of the mutant pcbAB gene, particularly one with a phenotype of increased enzyme activity, will result in a further enhancement in intracellular enzyme activity, which in turn may be accompanied by an increase in productivity of the antibiotics of both the cephalosporin and penicillin pathways.

The following examples are offered by way of illustration and not by way of limitation.

EXPERIMENTAL

General Methods

In all examples described herein, the experimental techniques mentioned are known in the art: these have been described e.g. in Maniatis et al., supra).

Deposits

The following organisms have been deposited with Centraal Bureau voor Schimmelcultures (CBS), Oosterstraat 1, 3742 SK Baarn, Netherlands:

E.coli WK6 containing plasmid pPCV01, Accession No. CBS 142.90, was deposited February 28, 1990;

Cosmid HM193, Accession No. CBS 179.89, was deposited on April 3, 1989 as a DNA sample (cosmid clone in E.coli unstable);

P.chrysogenum strain Wisconsin 54-1255 npe5 containing an ACV Synthetase gene mutation, Accession No. CBS 178.89, was deposited on April 3, 1989.

Example 1

Characterization of the pcbAB gene from P.chrysogenum

A. Isolation of the pcbAB gene

The isolation of DNA fragments encoding the ACV Synthetase enzyme is described in EP-A-357119. Cosmid HM193 contains one such DNA fragment. Using parts of cosmid clone HM193 as a probe, cosmid clones containing similar or overlapping inserts have been isolated, using techniques known in the art. The presence of the pcbAB gene on the DNA fragments has been demonstrated in various ways. The DNA fragments were able to complement a mutation in the gene encoding ACV Synthetase, which is present in the strain Wisconsin 54-1255 npe5, deposited as CBS 178.89. This is a mutant ACV Synthetase-negative P.chrysogenum strain derived from Wis54-1255. Restoration of penicillin production by complementation was accompanied by the reappearance of in vitro ACV Synthetase activity in cell-free extracts. Moreover, restoration of penicillin production was also accompanied by reappearance of a large protein on SDS-PAGE gels.

B. Localization of the pcbAB gene

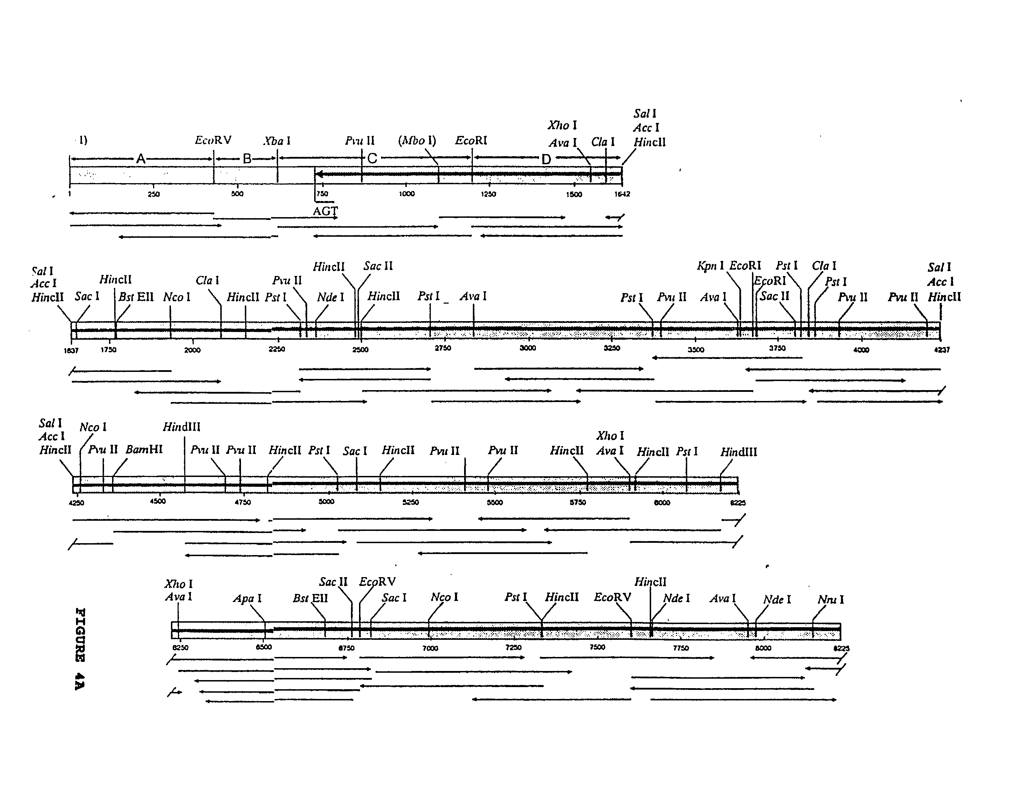

Northern hybridizations were performed using mRNA that was isolated from penicillin-producing cultures as described in EP-A-354624. The subcloned SalI fragments indicated in Figure 3 were used as

EP 0 444 758 A1

probes. Using probes I, II, III and IV a large mRNA ($\geq$ 10 x 10³ nucleotides) was detected in the Northern blot hybridizations. Probe V detected the mRNAs of the pcbC and penDE genes (about 1.5 x 10³ nucleotides). Using smaller probes (A-D and E-H in Figure 4), the positions of the putative 5'- and 3'-ends of the gene were located. The gene encoding the ACV Synthetase is present on the chromosome of P.chrysogenum as indicated by the shaded region in the schematic of Figure 3.

C. Polarity of the gene

The direction of transcription was determined by Northern hybridization of mRNA of P.chrysogenum using as a probe the following synthetic oligonucleotides that have been designed based on the nucleotide sequence surrounding the two HindIII sites located within the ACVS coding region (Figure 4).

```
AB1504 : 5'-CCC AGA CGC ACT TGA TCC TG-3'
AB1505 : 5'-GTC CCC GCT TGC GAC GAC TG-3'
AB1549 : 5'-CGG GAA TCA TCT GCG TAT C -3'
AB1550 : 5'-CGC GCT CAA AGG CCT GGT TC-3'
```

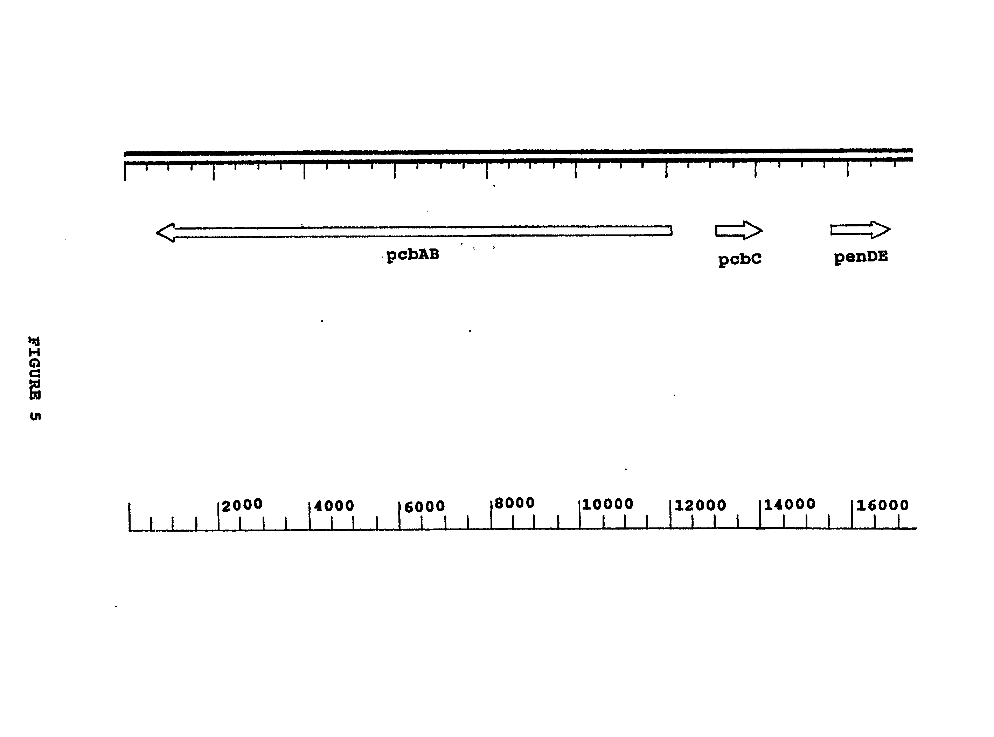

Only probes AB1549 and AB1550 hybridized to the ACV Synthetase mRNA and the deduced direction of transcription is indicated in Figure 5. The direction of transcription of the ACV Synthetase gene is in the opposite direction to the genes encoding IPNS and AT.

D. Determination of the nucleotide sequence

The nucleotide sequence of a DNA fragment contained in cosmid HM193 containing the ACV Synthetase gene was determined by the dideoxy method (Sanger et al., Proc. Natl. Acad. Sci. U.S.A. - (1977), 74:5463-5467) using the Sequenase system 2.0 (U.S. Biochemicals, Cleveland, OH). Double sequencing reactions with dGTP and dITP were used in some clones to avoid errors (Barnes et al., Methods in Enzymology (1983), 101:98-122). The sequencing strategy is given in Figure 4. The nucleotide sequence of the 12,363 nucleotide DNA fragment is given in Sequence listing 1. In this sequence a long open reading frame (ORF) of 11,337 nucleotides was found from which a protein sequence of 3778 amino acids was deduced (Sequence listing 1).

E. Determination of partial protein sequence of ACV Synthetase

To further confirm that the gene cloned is indeed the gene encoding the P.chrysogenum ACV Synthetase, amino acid sequences have been obtained from ACV Synthetase that has been purified from A.nidulans. The similarity between protein and nucleotide sequence data confirm the identity of the cloned gene.

ACV Synthetase was purified from A.nidulans, strain G-69, as has been described in Van Liempt et al. (supra).

The enzyme containing fractions from the DEAE column were applied to a Mono Q column on a FPLC apparatus (Pharmacia). ACV Synthetase was eluted using a 0-300 mM NaCl gradient in Tris/HCl buffer (pH 7.5). The peak fraction, having a protein concentration of 0.5 mg/ml was shown to contain almost pure (over 90%) ACV Synthetase; this was demonstrated by SDS-PAGE.

For digestion with protease, 1.25 ml (0.625 $\mu$g) of ACV Synthetase from the Mono Q fraction was incubated with 62.5 $\mu$l (6.3 $\mu$g) of Subtilisin (Sigma) for 60 min. at room temperature. The reaction was terminated by the addition of trichloroacetic acid (10% final concentration). The precipitated protein was recovered by centrifugation, dissolved in approximately 200 $\mu$l of Laemmli sample buffer, and the mixture was neutralized by the addition of 4M Tris. The protein was dissolved and incubated for 5 min. at 95° C.

A slab gel of 140 x 170 x 1.5 mm of polyacrylamide (separating gel 5% T), with a 3% stacking gel (Laemmli) was allowed to polymerize overnight at 4° C. The digested protein was applied in several slots, and electrophoresis was carried out with 0.02% thioglycolic acid in the upper buffer compartment. After the Bromophenol Blue marker had migrated up to 70% of the gel length, the electrophoresis was terminated and the proteins were transferred electrophoretically onto a PVDF-membrane (Immobilon) in a semidry blotting apparatus (Sartorius). The transfer buffer was 25 mM Tris/HCl (pH 8.5), 0.5 mM dithioerythritol.

11

After transfer (approximately 2 hrs.) the membrane was washed with water, stained in 0.5% Coomassie R 250, 50% Methanol for 5 min., destained in 50% Methanol, 10% Acetic Acid, washed with water and air dried.

From the complex pattern of protein bands present on the membrane, the bands that were the most pronounced and least contaminated with other nearby bands were excised. The amino acid sequence of the peptides in the excised bands was determined using a gas-phase sequenator (Applied Biosystems model 470a). The following sequence was determined (in this notation amino acids separated by a slash indicates ambiguity of interpretation at this position while an amino acid in parentheses indicates uncertainty in the interpretation; Xxx indicates the presence of an unidentified amino acid):

```
band 3: Asn Ala Asn Val Tyr Leu Ala Asn Ser Leu Gln Gln Gly
        Phe Val Tyr Gln Phe Leu Lys Asn Met Gly Asp/Arg Ser Gly/Trp
        Ala Asp/Tyr Asp/Val Met Gln Xxx Val (Thr) (Asp/Arg) Tyr


band 9: Gln Ser Val Gln Xxx Ale Lys Ser Val Ala Lys Phe Asp
        Leu Asn (Ala Thr) Ala Xxx (Glu) (Leu/Ser Asp/Gly Lys Ala)




band 12B: (Gln/Ser/Cys Gln Thr) Val Leu Gly Asp Ala Pro Leu
          Leu Pro Ile Gln (Thr/Gln His/Gln Phe)
```

F. Comparison of protein and nucleotide sequences

The amino acid sequences from Example 1E (A.nidulans) were compared with the deduced amino acid sequence from Example 1D (P.chrysogenum), which is shown in Sequence listing 1. For this comparison the MicroGenieTM 6.0 program (Beckman) was used.

The following results have been obtained:

```
aa band 3      1   N   A   N   V   Y   L   A   N   S   L   Q   Q   G   F   V
                   |   :   |   |   |   |   |   |   |   |   |   |   |   |   |
Pc ACVS      948   T   D   N   I   Y   L   A   N   S   L   Q   Q   G   F   V


                                           (D) *   (W)       (D) (D)
aa band 3     16   Y   Q   F   L   K   N   M   G  (R)  S  (E)  A  (Y) (Y)  M
                   |   :   |   |       |       |   :   |   :   |   :       |
Pc ACVS P    963   Y   H   Y   L   K   S   M   E   Q   S   D   A   Y   V   M


                               (D)
aa band 3     31   Q   X   V   T  (R)  Y
                   |   |   |   |   |   |
Pc ACVS P    978   Q   S   V   L   R   Y

Matches = 22 (25)    Mismatches      = 14 (11)
Length  = 36         Matches/Length  = 61.1 (69.4)  percent
```

```
aa band 9a     1   Q   S   V   Q   X   I   K   S   V   A   K   F   D   L   N
                   |   |   |   |       :   |   |   |   |   |   |   |   |   |
             2402  R   P   V   Q   P   V   D   S   V   A   K   F   D   L   N


                               (S) (E)
              16  [A   T   A   X   E  (L) (D)  K   A]
                   |   |       |   |   :
             2417  A   T   V   T   E   L   E   S   G

Matches = 12 (13)    Mismatches      = (7)
Length  = 24         Matches/Length  = 50.0 (54.2)  percent
```

```
                  (C)
                  (S)
aa band 12B    1  (Q)  Q   T   V   L   G   D   A   P   L   L   P   I   Q  (Q) (Q) (F)
                   |   |   :   |   :   |   |   |   |   |   |   |        :        |
Pc ACVS P    3110  Q   G   P   V   I   G   E   A   P   L   L   P   I   Q   D   W   F

Matches = 10 (11)    Mismatches      = 7 (6)
Length  = 17         Matches/Length  = 58.8 (64.5)  percent
```

|  =  Homologous residues
:  =  Similar residues (defined according to the Beckman standard: A replacement is defined conservative when both amino acids belong to one of the following sets: (A,S,T), (N,Q), (D,E), (I,L,M,V), (R,H,K), or (F,W,Y). MicroGenie™ Manual MG-IM-6.0, Dec. 1988).

The degree of homology found between A.nidulans and P.chrysogenum varies between 50 and 61%. This is very similar to the degree of homology between the IPNS proteins derived from both organisms (e.g. G. Cohen et al., Trends in Biotechnology (1990), 8:105-111); hence the protein data confirm the conclusion that the gene cloned is indeed the P.chrysogenum ACV Synthetase gene.

Example 2

Expression of the pcbAB gene from P.chrysogenum in P.chrysogenum

Synthesis of pPCV01:

This vector was derived from pBluescript II KS M13(+) (Stratagene, La Jolla, CA) and contains the

phleomycin resistance gene under control of the P.chrysogenum pgk promoter. It moreover contains a synthetic multiple cloning site, including unique SpeI and XbaI sites. The P.chrysogenum pgk gene has been isolated from a genomic cosmid library (EP-A-357119) using the corresponding gene of Saccharomyces cerevisiae (Dobson et al., Nucleic Acid Research (1982), 10:2625-2637) as a probe (Van Solingen et al., Nucleic Acid Research (1988), 16:11823). The sequence of part of the promoter is disclosed in EP-A-354624. The promoter and a small part of the coding region can be isolated as a 1.5 kb HindIII fragment.

Synthesis of PCV02:

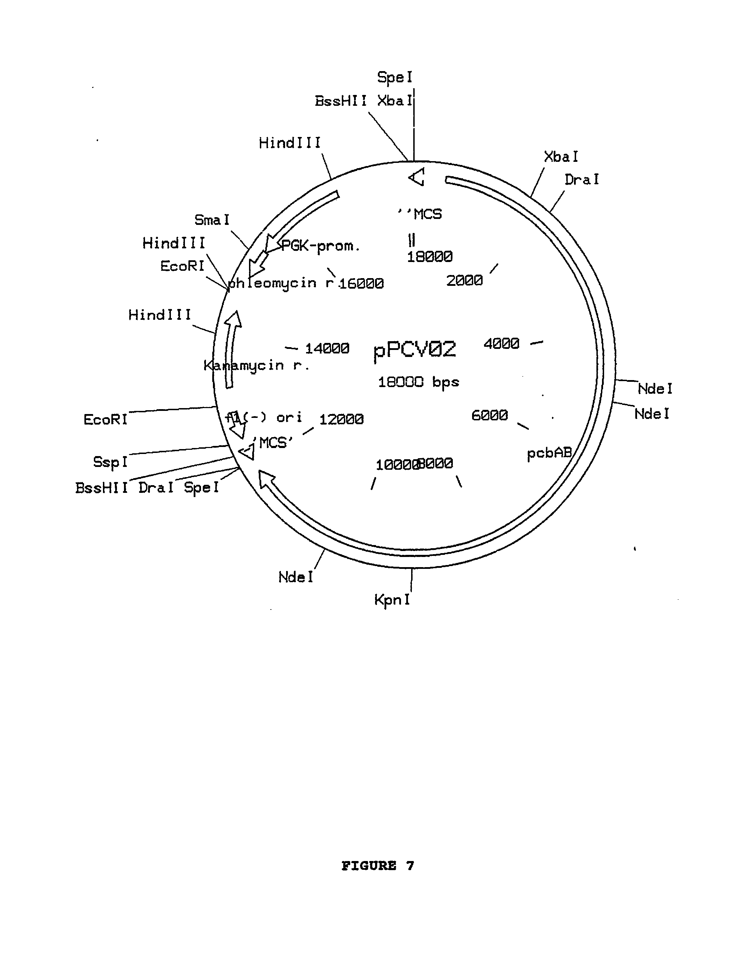

The pcbAB gene is isolated from cosmid clone HM193 as a $1.2 \times 10^4$ nucleotides SpeI fragment and was subcloned into the vector pPCV01, using the unique SpeI site. Conditions used were as disclosed in Maniatis et al. (supra). The resulting construct is named pPCV02 (Figure 7). The orientation of the gene in the vector has been determined by digestion with restriction enzymes.

Transformation

The plasmid pPCV02 has been transformed into P.chrysogenum Wis54-1255 npe5 (CBS 178.89) using the procedure described in EP-A-260762. Strain npe5 is a non-producing mutant of Wis45-1255; the npe phenotype is caused by the absence of ACV synthetase activity. Transformants have been selected for resistance against 30 μg/ml of phleomycin. Isolated transformants have been tested in a bioassay, as described in EP-A-354624, for a restoration of penicillin production. In a representative experiment penicillin production has been restored in 80% (8 out 10) of the pPCV02-transformants analysed; in transformants having received the vector pPCV01, without the ACV synthetase insert, a restoration of the penicillin production has not been demonstrated (0 out of 26).

The construct pPCV02 is also transformed into wild type P.chrysogenum. Selected transformants are assayed for an increased ACV synthetase activity, using cell-free extracts as described in EP-A-357119, or for an increased penicillin productivity, using shake flask experiments as have been described in EP-A-354624.

Example 3

Expression of the pcbAB gene from P.chrysogenum under control of the P.chrysogenum pgk promoter

Transcription of the pcbAB gene is subject to glucose repression

mRNA preparations, isolated from Penicillium cultures grown on either glucose- or lactose-containing media (EP-A-354624, are transferred to gene Screen-plus (NEN/ DuPont) and hybridized with the $1.5 \times 10^3$ nucleotides HindIII fragment, which is internal to the pcbAB gene (Figure 4). In glucose-grown cultures, no pcbAB mRNA is detected, while in the lactose grown cultures, a large mRNA ($>10 \times 10^3$ nucleotides) is detected.

Construction of pPCV03

The region surrounding the ATG start codon of the pcbAB gene is isolated as a $1.7 \times 10^3$ nucleotides SalI-DraI fragment. The vector pTZ18R (US Biochemical Corporation, Cleveland, OH) is digested with SalI and SmaI restriction enzymes. The digested vector and the $1.7 \times 10^3$ fragment are ligated. A construct containing the pTZ18R vector bearing the $1.7 \times 10^3$ fragment insert is isolated. Into this construct, the P.chrysogenum pgk promoter is ligated as a $1.5 \times 10^3$ nucleotides HindIII fragment (EP-A-354624). A construct containing the pgk promoter in the desired orientation (same polarity as the pcbAB fragment) is isolated. From this construct single stranded DNA is isolated by superinfection with the helper phage M13K07, (U.S. Biochemical Corporation, Cleveland, OH) using techniques known in the art or as prescribed by the supplier of the pTZ cloning vector. By in vitro mutagenesis using a synthetic oligonucleotide having the following sequence:

5'-TGG CTT CAG TTG AGT CAT ATG GGT AGT TAA TGG TAT-3', a DNA fragment containing the mature pgk region and the region upstream of the pcbAB ATG is looped out. This mutagenesis introduces an NdeI site at the position of the ATG (underlined in the oligonucleotide). The construct is named pTZpgk::acvsi. This construct is digested with HindIII and XbaI and the promoter-gene fusion is isolated on a DNA

14

fragment of 2.9 x 10³ nucleotides as described by Maniatis et al. (supra). This fragment is ligated with XbaI-linearized pPCV02, the remaining HindIII-XbaI sticky ends are filled in with T4 DNA polymerase and the construct is circularized by the addition of ligase.

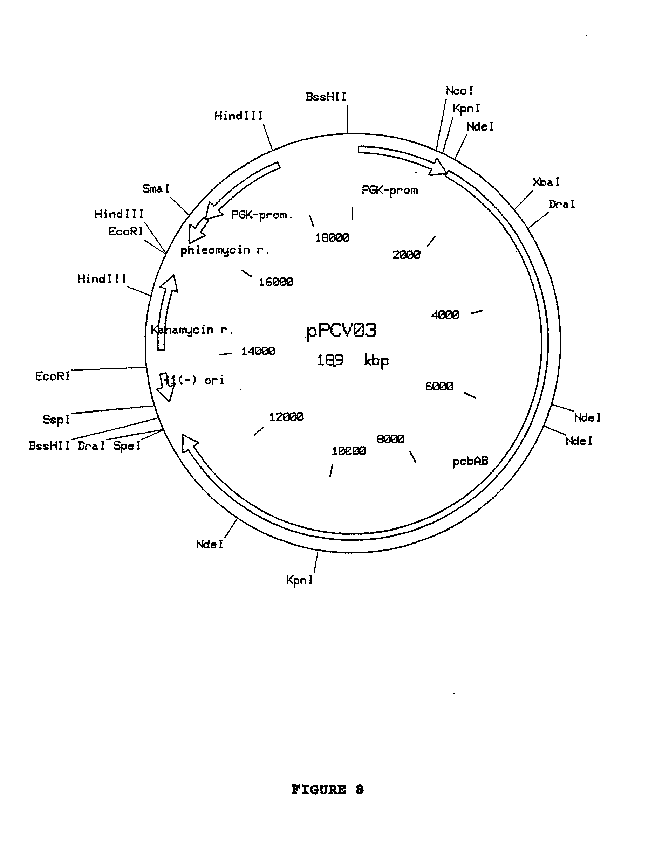

The resulting construct, pPCV03 (Figure 8), is isolated as follows. The ligation mixture is transformed into E.coli HB101 (ATCC 33694) using standard techniques. Plasmid DNA is isolated from several transformants and analysed by restriction enzyme-digestion and agarose gel electrophoresis. Transformants containing the correct DNA constructs are grown on large scale (500 ml) and plasmid DNA is isolated using methods as disclosed in Maniatis et al. (supra) and transformed into P.chrysogenum. Expression of the pcbAB gene, enzyme activity and penicillin production of transformants is analyzed after growth in shake flasks on both glucose- and lactose containing media; the data obtained are compared with those obtained for transformants containing pPCV02. In contrast to transformants containing pPCV02, transformants containing pPCV03 express the pcbAB gene in glucose-containing media: both an ACV Synthetase-specific mRNA and ACV Synthetase enzyme activity are detected.

Example 4

Expression of the pcbAB gene in E.coli

For efficient production of ACV Synthetase enzyme in E.coli it is necessary to place the pcbAB gene under control of a promoter which allows efficient gene expression in E.coli. Examples of such efficient promoters are the trp promoter, the lac promoter and the tac promoter. In this example the trp promoter is described, but it will be obvious to those skilled in the art that the experiments can be easily repeated with the lac and tac promoters, leading to similar results. If desired, a runaway replicon can·be included in the construct; this will allow for controlled amplification of the plasmid copy number after a temperature shift.

Synthesis of pMA-ACVS:

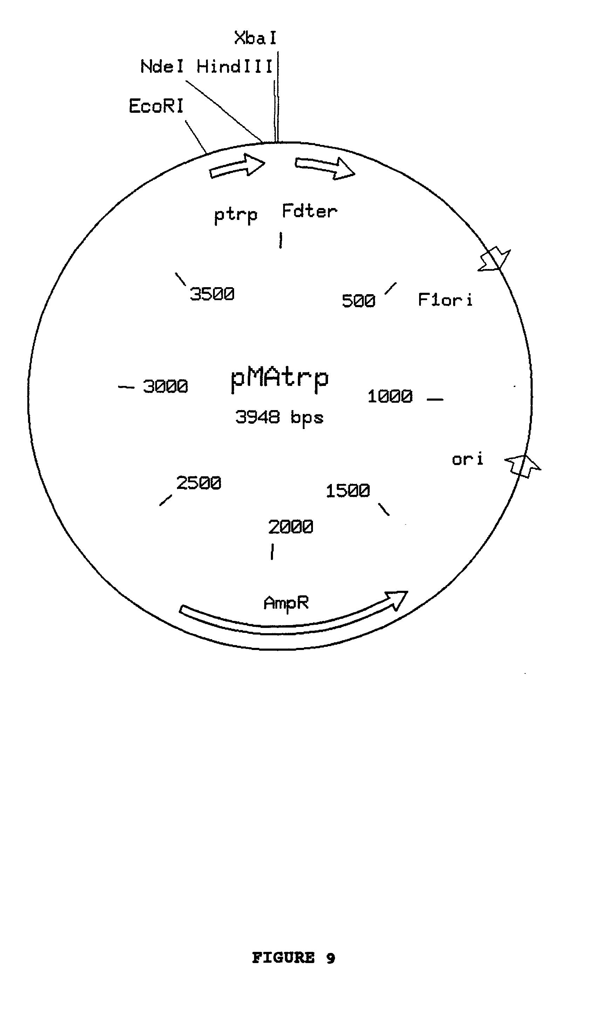

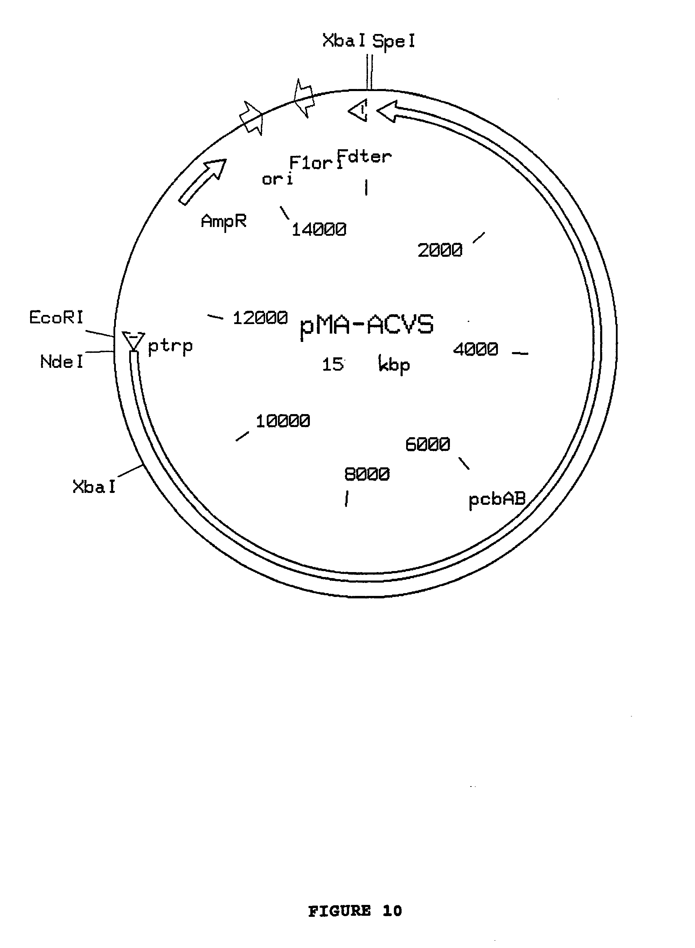

pMAtrp (Figure 9), having the trp promoter region between -113 and the ATG transcription start flanked by an NdeI site at the position of the ATG (Sommerville, supra), is digested with SmaI and NdeI. From pPCV03 a 1.5 x 10³ nucleotides NdeI-DraI fragment, containing the 5'-part of the pcbAB gene, is isolated and ligated into the SmaI, NdeI digested pMAtrp. Into the XbaI sites of the resulting construct, the 3'-part of the pcbAB gene is inserted as a 1.0 x 10⁴ nucleotides XbaI fragment isolated from HM193 or pPCV02. Constructs are selected for the correct orientation of the inserted XbaI fragment; the construct containing the fragment in the correct orientation is named pMA-ACV Synthetase (Figure 10). Orientation is determined by digestion with various restriction enzymes.

pMA-ACV Synthetase is isolated and used to transform a suitable E.coli host, such as, for example E.coli HB101, E.coli C600 or E.coli JM101. Transformants are analyzed by determination of ACV Synthetase activity in cell-free extracts, by electrophoresis of cell-free extracts in 5% polyacrylamide gels (SDS-PAGE) and by immunoblotting, using a polyclonal antiserum which had been raised against purified ACV Synthetase.

Example 5

Expression of the pcbAB gene from P.chrysogenum in Streptomyces lividans

For expression in a Streptomyces host, several options are available. S.lividans is a preferred host because of the ease of transformation of this host, as compared to several other Streptomycetes.

Expression of the P.chrysogenum ACV Synthetase gene under control of the aph promoter

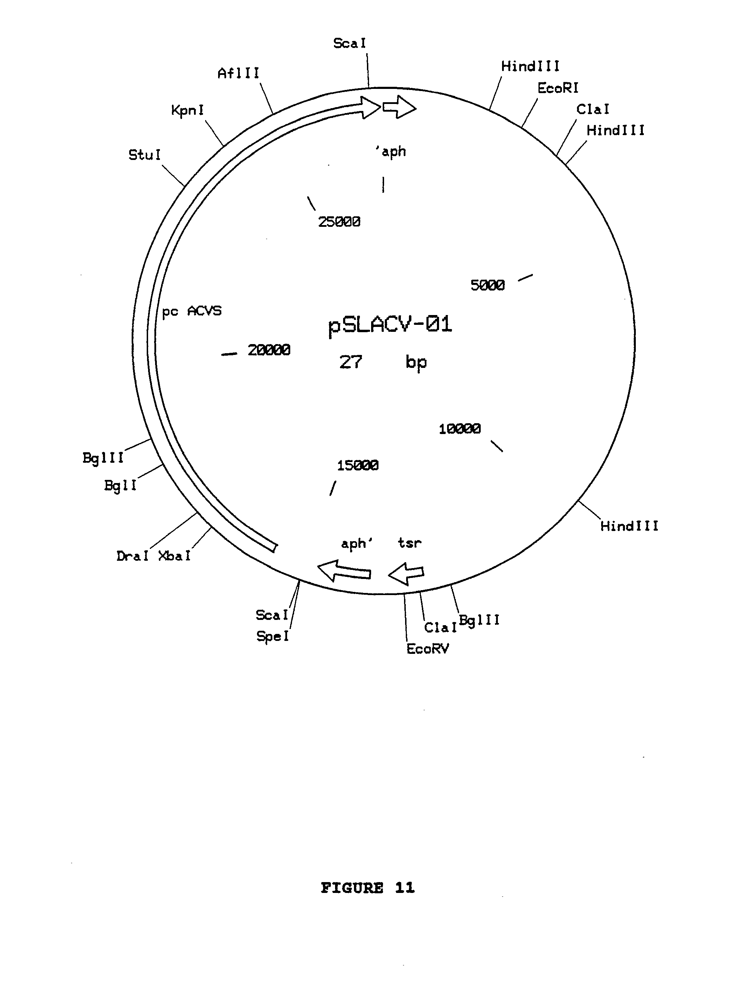

The P.chrysogenum ACV Synthetase gene is isolated as the 1.2 x 10⁴ nucleotides SpeI fragment described in Example 2. The sticky ends of this fragment are made blunt by treatment with T4 DNA polymerase using the procedures known in the art. The vector pIJ61 has been described by C.J. Thompson et al., Gene (1982), 20:51-62; reviewed by: D.A. Hopwood et al. (supra); the vector can be obtained from D.A. Hopwood. The vector was digested with BamHI, and the ends were made blunt using T4 DNA polymerase. Subsequently, the blunt-ended 1.2 x 10⁴ nucleotides SpeI fragment is inserted into the blunt-ended BamHI site via ligation, and the mixture is used to transform S.lividans 66, by the method of Hopwood et al. (supra). Transformants are selected for resistance to thiostreptone (50 μg/ml) and are

subsequently analyzed for the orientation of the pcbAB gene within the aph gene. A construct having the pcbAB gene in the same orientation as the aph gene is named pSLACV-01 (Figure 11).

Selected transformants containing the plasmid pSLACV-01 were cultured as described in Chen et al. (supra). Cell-free extracts are prepared and analyzed by SDS-PAGE or immuno-blotting for the presence of a large (>250 kDa) protein; the ACV Synthetase activity in the extracts is also determined using the procedure as described by Van Liempt (supra).

Expression of the P.chrysogenum pcbAB gene in S.lividans under control of the tyrosinase promoter

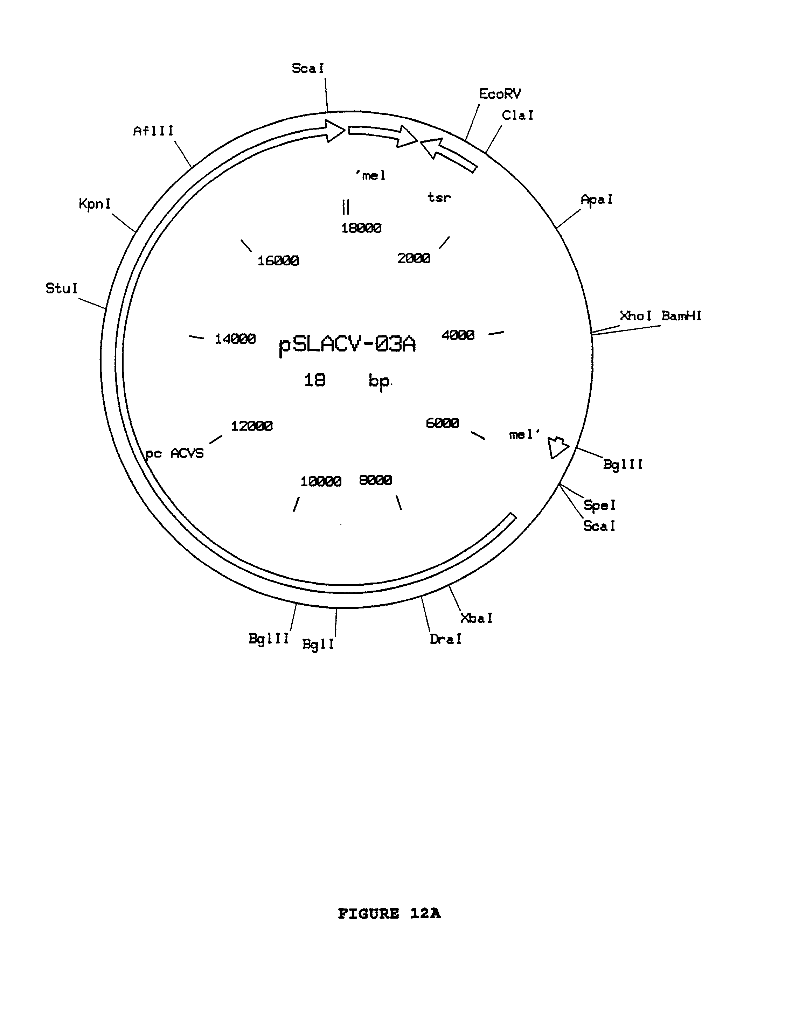

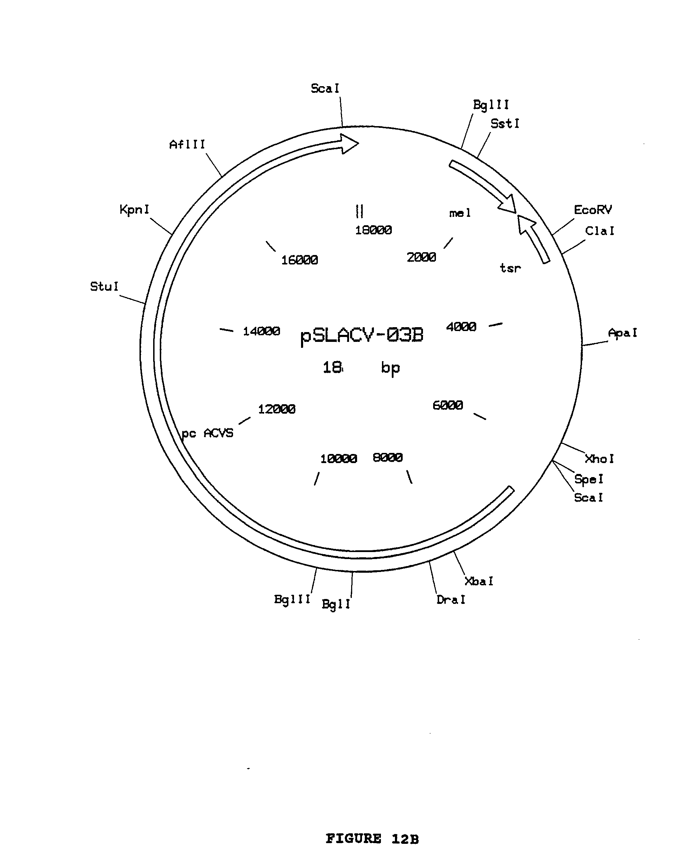

The vector pIJ702 is digested with either BglII or SstI. pIJ702 is described by E. Katz et al., Journ. Gen. Microbiol. (1983), 129:2703-2714; reviewed in: D.A. Hopwood et al., supra; obtainable from D.A. Hopwood. BglII digested pIJ702 was made blunt-ended by treatment with T4 DNA polymerase; SstI digested pIJ702 is treated with Mung bean nuclease in order to obtain blunt ends. The blunt-ended SpeI fragment containing the P.chrysogenum pcbAB gene as described hereinabove is inserted into both blunt-ended vectors via ligation. Thiostreptone resistant transformants are screened for a melanin-negative phenotype by application of a soft agar overlay containing tyrosine (0.1 mM) as described in Hopwood et al. (supra). Melanin-negative transformants contained an interrupted tyrosinase gene (by insertion of the ACV Synthetase gene) and their colonies remained colorless upon addition of tyrosine, while wild-type colonies turned brown. Analysis by restriction enzyme digestion and agarose gel electrophoresis for the correct orientation of the inserts yielded plasmids pSLACV-03A (SstI site; Figure 12A) and pSLACV-03B (BglII site; Figure 12B).

Analysis of transformants is as described in Example 5a. Induction of the tyrosinase promoter is established by the addition of methionine (10 mM) to the culture medium.

Example 6

Modification of the ACVS protein by exchange of domains: construction of hybrid proteins

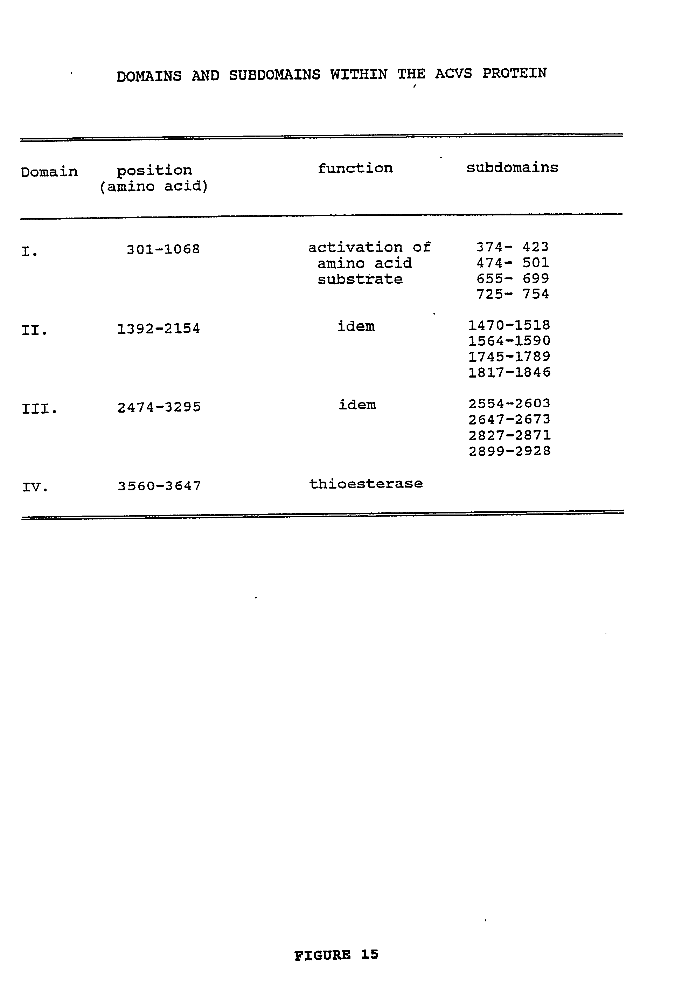

From the nucleotide sequence of the pcbAB gene an amino acid sequence has been deduced, as indicated in Sequence listing 1. Upon matrix comparison of this protein sequence with itself, three distinct regions of homolcgy are found: these regions are defined herein as domains. These domains are located between amino acids 301 and 1068 (domain I), 1392 and 2154 (domain II) and between amino acids 2474 and 3295 (Domain III; Figures 13 and 15). Similar domains were found in the pcbAB gene of A.chrysogenum. Within these domains, several even more conserved elements can be distinguished. A summary is given in Figure 15. Since the ACVS enzyme synthesizes a tripeptide, which requires the activation of three amino acids, a role of these three domains in the amino acid activation reactions seems likely. Therefore, the said domains are candidate regions for in vitro mutagenesis.

Comparison of the deduced amino acid sequence with the protein sequences known for other multifunctional enzymes reveals a significant homology with the Bacillus brevis tyrocidin synthetase I (hereinafter referred to as TYI; Weckermann et al., Nucleic Acids Research (1988), 16:11841) and gramicidin synthetase I (hereinafter referred to as GSI; Krätzschmar et al., supra; compare Figure 13). Since both TYI and GSI are involved in activation and racemisation of the amino acid phenylalanine, this homology supports the notion that these conserved-sequences may represent centers involved in ATP-mediated activation of amino acids. If the mechanism of peptide synthesis by ACVS or Gramicidin Synthetase is comparable, the expected order of the domains in the enzyme is in the same order as that of the amino acids in the tripeptide (Krätzschmar, supra).

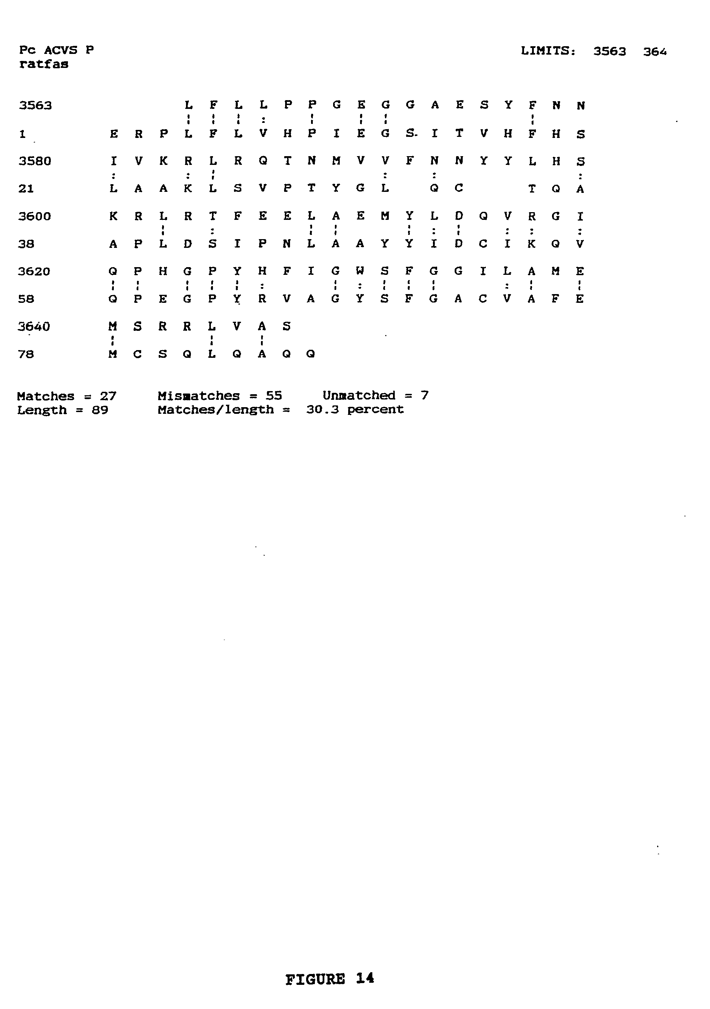

Upon comparison of the ACVS protein sequence with other known protein sequences of large enzymes, a significant homology is found with the Fatty Acid Synthetases (long chain) from rat and chicken (Figure 14; M. Schweizer et al., Nucleic Acids Research (1989), 17:567-586; Z. Yuan et al., PNAS (1988), 85:6328-6331). The homology is found between the COOH-terminal part of the ACVS protein (domain IV in Figure 15) and the thioesterase domain of the long chain Fatty Acid Synthetase proteins. Even the active site of the thioesterase, viz. G.X.S.X.G (e.g. Krätzschmar, supra), is present in the ACVS protein sequence. A similar homology has been described for a subunit of gramicidin Synthetase; in this case the homology is found between the grsT subunit and the type II fatty acid synthetases. The demonstration of the said homology suggests that ACVS most probably also contains within its structure the ability to release the tripeptide, once it has been formed and bound to the enzyme by thioester bond formation, by the action of its thioesterase domain. This activity may form another target for in vitro mutagenesis, e.g. by increasing the efficiency of the release of the tripeptide, in case this part of the reaction is the rate determining step. However, in experiments designed to change substrate specificity, this domain preferably remains unchanged.

The functional domains of the ACVS protein being identified, it now is possible to construct modified peptide synthetases by the exchange of functional domains. This can be achieved e.g. by interchanging the three domains that have been identified within the ACVS protein. An alternative way is the exchange of ACVS specific domains with domains from other proteins known to have a similar function. Selected domains can be isolated by restriction enzyme digestion of clones containing the genes encoding the said proteins or domains thereof. However, suitable restriction sites at useful positions are seldom encountered. Therefore, the Polymerase Chain Reaction offers a good alternative for the isolation of suitable DNA fragments. In general, DNA fragments containing functional domains are prepared as follows: for each domain two oligonucleotides are designed, one starting at the N-terminal end of the selected domain, in the direction of the C-terminal end. The other oligonucleotide is derived from the C-terminal end of the domain and is designed in the opposite direction; consequently this oligonucleotide is derived from the other DNA strand. At the 5'-end of each oligonucleotide a suitable restriction site can be included in the oligonucleotide, in order to facilitate ligation of the domains after amplification. The DNA fragments between the two oligonucleotides are amplified using the polymerase chain reaction, thereby following the protocols known in the art (described e.g. in 'PCR-Technology', supra). Preferably, Tag DNA polymerase is used for the amplification reaction. The method is exemplified by exchanging domain I of ACVS by the phenylalanine-activating domain of GSI. For the in vitro synthesis of the GSI domain the following oligonucleotides are designed:

A 5'-CGG CAT ATG TTA AAC AGT TCT AAA AGT ATA -3'

B 5'-GCA AGA TCT TCT AGG AAT AAT TCG CT -3'.

Oligo A generates an NdeI site at the 5'-end of the DNA fragment encoding the domain (underlined) and oligo B generates a BglII site at the 3'-end (underlined). The oligo's are used in a PCR reaction, with total DNA isolated from Bacillus brevis ATCC 9999 as the template. The desired DNA fragment encoding the domain is isolated as a $2.4 \times 10^3$ nucleotides DNA fragment. Sticky ends are generated by digestion with NdeI and BglII. The fragment is inserted in pMA-ACVS (Example 3) which has been partially digested with NdeI and with BglII. The ligation mixture is transformed into E.coli, and transformants are analyzed for the desired enzyme activity. To this end, cell-free extracts of transformants are analyzed for the presence of an new tripeptide, using the method that has been described before (M.J. Lopez-Nieto et al., Appl. Microbiol. Biotechnol. (1985), 22:343-351). Alternatively, cell-free extracts of transformants are used for the in vitro synthesis of tripeptide, thereby using phenylalanine, α-aminoadipic acid, cysteine and valine as the substrates. Tripeptides formed are analyzed as described before (M.J. Lopez-Nieto et al., supra) and are compared with chemically synthesized standards.

Alternatively, the GSI domain is inserted into pPCV03, the resulting construct is transformed into P.chrysogenum npe5 and transformants are screened for the presence of new tripeptides as described hereinbefore.

Example 7

Modification of the substrate specificity of ACVS

To modify the substrate specificity of ACVS, in vitro mutagenesis is performed using the methods known in the art. As targets for the mutagenesis are used the domains I, II or III or subdomains thereof, as specified in Figure 15. Domains are selected either at random, or, preferably, by their known activity for activation of either one of the fermentable or known amino acid substrates to be replaced to form an new tripeptide.

After mutagenesis, the mutated constructs are transformed to e.g. P.chrysogenum npe5, an ACVS-negative strain which has been deposited as CBS 178.79. P.chrysogenum transformants are screened for the production of antibiotic using the microbiological plate assay described in EP-A-354624; selected transformants are subsequently analyzed for the formation of new tripeptides or β-lactam antibiotics as described hereinbefore.

For a mass screen of transformants, the preferred hosts are E.coli or, alternatively, P.chrysogenum npe5 in which the pcbC gene has been inactivated by mutagenesis or gene replacement, since strains lacking IPNS are known to accumulate tripeptide.

The tripeptides formed in the bacterial or fungal transformants are subsequently analyzed by ELISA, using antibodies that have been raised against the desired -chemically synthesized- tripeptides. Preferably, the transformants are grown in microwell plates and the ELISA assay is automated using e.g. the tecan laboratory robot system, e.g. type rsp8051 (Tecan).

The examples demonstrate that the ACV Synthetase enzyme activities reside on a single gene in P.chrysogenum and that elevated activity of this enzyme can be obtained in host cells using the recombinant compositions of the instant invention. Furthermore, heterologous expression of ACV Synthetase has been demonstrated, which provides a means of using more efficient and robust hosts for the commercial production of ACV Synthetase and its secondary metabolites. Thirdly, mutant ACV Synthetases have been designed, with changed substrate specificities or with changed properties that make the enzyme more suited for the in vivo or in vitro synthesis of new tripeptide precursors to β-lactam antibiotics. Finally, the availability of large amounts of the ACV Synthetase-enzyme, e.g. by expression of the gene in a suitable host, will allow for better prospects for commercial application of in vitro synthesis of β-lactam antibiotics and their precursors.

All publications and patent applications cited in this specification are herein incorporated by reference as if each individual publication or patent application were specifically and individually indicated to be incorporated by reference.

The invention now being fully described, it will be apparent to one of ordinary skill in the art that many changes and modifications can be made thereto without departing from the spirit or scope of the appended claims.

**Sequence Listing No. 1**

SEQ. No.: 1
SEQUENCE TYPE : Nucleotide with corresponding protein
SEQUENCE LENGTH: 12364 base pairs
STRANDEDNESS: single
TOPOLOGY: linear
MOLECULE TYPE: genomic
ORGANISM: Penicillium chrysogenum
FEATURES: from 264 to 11600 bp peptide
PROPERTIES: gene encoding ACV Synthetase

```
GTCGACAGTT GACAGAGCCA ATGGCATCGG ATCTGCCCTA GACCGTGCTA GACGAAAGTC      60
TCCATCTTGT CTGCGGGCAG TTCTTCAGTC GCCCAGATTC TCGATGGAGA TTGGCCAGGT     120
CAGCCATATA TACCCTGCAA TGGCAGACCA ATGCAGCAGG CCCAGTATAA GGAATTCCCC     180
TCGAGCTTGT CTGTGATTGC GTTTTTTCTA ACACTTGTTG TTGCATCCGA TCCGTCGCTA     240
CCAATTATTG GTCATTGACA GAC ATG ACT CAA CTG AAG CCA CCG AAC GGA ACC     293
                       Met Thr Gln Leu Lys Pro Pro Asn Gly Thr
                        1                   5                   10

ACG CCG ATA GGC TTC TCG GCC ACT ACA TCC CTG AAC GCC AGT GGG AGC       341
Thr Pro Ile Gly Phe Ser Ala Thr Thr Ser Leu Asn Ala Ser Gly Ser
                15                  20                  25

TCG AGT GTG AAA AAT GGG ACC ATC AAA CCC AGC AAT GGC ATC TTC AAG       389
Ser Ser Val Lys Asn Gly Thr Ile Lys Pro Ser Asn Gly Ile Phe Lys
                30                  35                  40

CCC AGC ACT AGG GAC ACC ATG GAC CCT TGC AGT GGG AAT GCG GCC GAT       437
Pro Ser Thr Arg Asp Thr Met Asp Pro Cys Ser Gly Asn Ala Ala Asp
            45                  50                  55

GGC AGT ATC CGC GTC CGT TTC CGT GGA GGA ATC GAA CGG TGG AAG GAG       485
Gly Ser Ile Arg Val Arg Phe Arg Gly Gly Ile Glu Arg Trp Lys Glu
        60                  65                  70

TGC GTC AAC CAG GTC CCC GAG CGC TGC GAC CTG AGT GGT CTG ACA ACC       533
Cys Val Asn Gln Val Pro Glu Arg Cys Asp Leu Ser Gly Leu Thr Thr
    75                  80                  85                  90

GAC TCC ACG CGA TAT CAG CTC GCA TCG ACT GGG TTC GGT GAC GCG AGC       581
Asp Ser Thr Arg Tyr Gln Leu Ala Ser Thr Gly Phe Gly Asp Ala Ser
                95                  100                 105

GCT GCG TAC CAG GAG CGC TTG ATG ACG GTC CCT GTT GAC GTA CAT GCC       629
Ala Ala Tyr Gln Glu Arg Leu Met Thr Val Pro Val Asp Val His Ala
            110                 115                 120

GCG CTC CAA GAG CTG TGC CTA GAA CGC CGT GTG AGC GTG GGA TCC GTC       677
Ala Leu Gln Glu Leu Cys Leu Glu Arg Arg Val Ser Val Gly Ser Val
        125                 130                 135

ATT AAT TTC TCC GTG CAC CAG ATG CTG AAA GGG TTT GGA AAT GGC ACA       725
Ile Asn Phe Ser Val His Gln Met Leu Lys Gly Phe Gly Asn Gly Thr
    140                 145                 150
```

**Sequence Listing No. 1 (continued)**

```
CAC ACT ATC ACC GCC TCT CTG CAC CGT GAG CAG AAT TTG CAG AAT TCT      773
His Thr Ile Thr Ala Ser Leu His Arg Glu Gln Asn Leu Gln Asn Ser
155                 160             165             170

TCG CCA TCC TGG GTA GTC TCC CCC ACA ATC GTC ACC CAT GAG AAC AGA      821
Ser Pro Ser Trp Val Val Ser Pro Thr Ile Val Thr His Glu Asn Arg
            175             180             185

GAC GGA TGG TCC GTC GCG CAG GCG GTC GAG AGT ATC GAA GCG GCG CGC      869
Asp Gly Trp Ser Val Ala Gln Ala Val Glu Ser Ile Glu Ala Ala Arg
            190             195             200

GGT TCC GAG AAG GAG TCA GTG ACT GCG ATT GAC TCC GCG TCA AGT CTC      917
Gly Ser Glu Lys Glu Ser Val Thr Ala Ile Asp Ser Ala Ser Ser Leu
            205             210             215

GTG AAA ATG GGG TTA TTT GAC TTA CTC GTC AGC TTT GTC GAT GCA GAC      965
Val Lys Met Gly Leu Phe Asp Leu Leu Val Ser Phe Val Asp Ala Asp
    220             225             230

GAT GCT CGT ATT CCA TGT TTC GAC TTT CCC CTC GCA GTG ATA GTG CGT     1013
Asp Ala Arg Ile Pro Cys Phe Asp Phe Pro Leu Ala Val Ile Val Arg
235             240             245             250

GAG TGT GAT GCC AAC CTC TCG CTG ACT CTG CGT TTC TCC GAC TGT CTC     1061
Glu Cys Asp Ala Asn Leu Ser Leu Thr Leu Arg Phe Ser Asp Cys Leu
                255             260             265

TTC AAC GAG GAG ACG ATA TGC AAT TTT ACC GAT GCC CTA AAC ATC TTG     1109
Phe Asn Glu Glu Thr Ile Cys Asn Phe Thr Asp Ala Leu Asn Ile Leu
                270             275             280

CTC GCC GAA GCA GTG ATA GGA AGA GTG ACC CCG GTT GCC GAT ATC GAA     1157
Leu Ala Glu Ala Val Ile Gly Arg Val Thr Pro Val Ala Asp Ile Glu
            285             290             295

CTA CTA TCC GCG GAG CAG AAG CAG CAG CTG GAA GAG TGG AAC AAC ACG     1205
Leu Leu Ser Ala Glu Gln Lys Gln Gln Leu Glu Glu Trp Asn Asn Thr
    300             305             310

GAT GGC GAG TAC CCT TCA TCA AAG CGA CTG CAC CAT CTC ATT GAA GAG     1253
Asp Gly Glu Tyr Pro Ser Ser Lys Arg Leu His His Leu Ile Glu Glu
315             320             325             330

GTG GTT GAA CGG CAT GAA GAC AAA ATA GCC GTT GTC TGC GAC GAG CGA     1301
Val Val Glu Arg His Glu Asp Lys Ile Ala Val Val Cys Asp Glu Arg
            335             340             345

GAG CTC ACT TAC GGC GAG CTC AAT GCC CAA GGC AAC AGC CTC GCA CGC     1349
Glu Leu Thr Tyr Gly Glu Leu Asn Ala Gln Gly Asn Ser Leu Ala Arg
            350             355             360

TAT CTC CGT TCC ATT GGT ATC CTG CCC GAG CAG CTA GTC GCA TTG TTT     1397
Tyr Leu Arg Ser Ile Gly Ile Leu Pro Glu Gln Leu Val Ala Leu Phe
    365             370             375
```

## Sequence Listing No. 1 (continued)

```
CTA GAT AAG AGC GAG AAG CTC ATT GTT ACC ATC CTC GGC GTG TGG AAA        1445
Leu Asp Lys Ser Glu Lys Leu Ile Val Thr Ile Leu Gly Val Trp Lys
    380                 385                 390

TCC GGC GCC GCC TAC GTG CCC ATC GAC CCG ACT TAT CCG GAT GAG CGA        1493
Ser Gly Ala Ala Tyr Val Pro Ile Asp Pro Thr Tyr Pro Asp Glu Arg
395                 400                 405                 410

GTG CGC TTC GTG CTG GAT GAC ACC AAG GCA CGG GCC ATC ATC GCC AGT        1541
Val Arg Phe Val Leu Asp Asp Thr Lys Ala Arg Ala Ile Ile Ala Ser
                415                 420                 425

AAT CAA CAT GTG GAG AGG CTC CAG CGA GAG GTC ATC GGC GAT AGA AAC        1589
Asn Gln His Val Glu Arg Leu Gln Arg Glu Val Ile Gly Asp Arg Asn
                430                 435                 440

CTA TGC ATT ATC CGT CTG GAG CCC TTG TTG GCC TCC CTT GCT CAG GAT        1637
Leu Cys Ile Ile Arg Leu Glu Pro Leu Leu Ala Ser Leu Ala Gln Asp
            445                 450                 455

TCC TCA AAA TTC CCC GCG CAT AAC TTG GAC GAC CTA CCC CTC ACA AGC        1685
Ser Ser Lys Phe Pro Ala His Asn Leu Asp Asp Leu Pro Leu Thr Ser
        460                 465                 470

CAG CAG CTC GCC TAT GTG ACT TAC ACC TCT GGG ACC ACT GGT TTC CCA        1733
Gln Gln Leu Ala Tyr Val Thr Tyr Thr Ser Gly Thr Thr Gly Phe Pro
475                 480                 485                 490

AAG GGC ATA TTT AAA CAA CAC ACC AAT GTG GTG AAC AGT ATT ACC GAC        1781
Lys Gly Ile Phe Lys Gln His Thr Asn Val Val Asn Ser Ile Thr Asp
                495                 500                 505

CTG TCT GCA AGG TAC GGG GTG GCC GGG CAG CAC CAC GAA GCC ATT CTG        1829
Leu Ser Ala Arg Tyr Gly Val Ala Gly Gln His His Glu Ala Ile Leu
                510                 515                 520

CTT TTC TCG GCC TGC GTG TTC GAG CCG TTC GTT CGA CAG ACG CTC ATG        1877
Leu Phe Ser Ala Cys Val Phe Glu Pro Phe Val Arg Gln Thr Leu Met
            525                 530                 535

GCA CTC GTG AAT GGC CAT CTC CTC GCA GTT ATC AAT GAC GTG GAA AAA        1925
Ala Leu Val Asn Gly His Leu Leu Ala Val Ile Asn Asp Val Glu Lys
        540                 545                 550

TAT GAT GCC GAT ACG CTC CTG CCG TTC ATA CGC AGA CAC AGC ATC ACC        1973
Tyr Asp Ala Asp Thr Leu Leu Pro Phe Ile Arg Arg His Ser Ile Thr
555                 560                 565                 570

TAC CTC AAT GGT ACT GCC TCT GTC TTG CAA GAG TAC GAC TTT TCC GAC        2021
Tyr Leu Asn Gly Thr Ala Ser Val Leu Gln Glu Tyr Asp Phe Ser Asp
                575                 580                 585

TGC CCA TCA CTG AAT CGG ATA ATC CTG GTG GGT GAG AAC CTG ACA GAA        2069
Cys Pro Ser Leu Asn Arg Ile Ile Leu Val Gly Glu Asn Leu Thr Glu
        590                 595                 600
```

21

**Sequence Listing No. 1 (continued)**

```
GCC CGG TAT CTG GCG CTG CGC CAG CGG TTC AAG AAT CGC ATC CTC AAC        2117
Ala Arg Tyr Leu Ala Leu Arg Gln Arg Phe Lys Asn Arg Ile Leu Asn
        605                 610                 615

GAG TAT GGT TTT ACC GAG TCA GCC TTT GTA ACG GCC CTC AAG ATT TTC        2165
Glu Tyr Gly Phe Thr Glu Ser Ala Phe Val Thr Ala Leu Lys Ile Phe
        620                 625                 630

GAC CCG GAG TCG ACC CGT AAG GAC ACG AGT CTG GGG AGA CCG GTG CGC        2213
Asp Pro Glu Ser Thr Arg Lys Asp Thr Ser Leu Gly Arg Pro Val Arg
635                 640                 645                 650

AAC GTC AAG TGC TAC ATC CTC AAT CCA TCC CTT AAA CGT GTC CCG ATT        2261
Asn Val Lys Cys Tyr Ile Leu Asn Pro Ser Leu Lys Arg Val Pro Ile
            655                 660                 665

GGA GCT ACG GGT GAG TTG CAT ATC GGA GGG TTG GGC ATT TCC AAG GGA        2309
Gly Ala Thr Gly Glu Leu His Ile Gly Gly Leu Gly Ile Ser Lys Gly
            670                 675                 680

TAC CTC AAC CGC CCC GAA CTC ACG CCG CAC CGC TTC ATT CCC AAC CCC        2357
Tyr Leu Asn Arg Pro Glu Leu Thr Pro His Arg Phe Ile Pro Asn Pro
            685                 690                 695

TTC CAA ACG GAT TGC GAG AAG CAG CTC GGG ATC AAC AGC TTG ATG TAC        2405
Phe Gln Thr Asp Cys Glu Lys Gln Leu Gly Ile Asn Ser Leu Met Tyr
        700                 705                 710

AAG ACC GGT GAC CTG GCC CGC TGG CTT CCG AAC GGC GAG GTT GAG TAT        2453
Lys Thr Gly Asp Leu Ala Arg Trp Leu Pro Asn Gly Glu Val Glu Tyr
715                 720                 725                 730

CTC GGA CGC GCA GAT TTC CAG ATC AAA CTG CGA GGT ATT CGA ATT GAA        2501
Leu Gly Arg Ala Asp Phe Gln Ile Lys Leu Arg Gly Ile Arg Ile Glu
            735                 740                 745

CCT GGT GAA ATT GAG ACG ATG CTG GCT ATG TAC CCT AGG GTC CGG ACC        2549
Pro Gly Glu Ile Glu Thr Met Leu Ala Met Tyr Pro Arg Val Arg Thr
            750                 755                 760

AGT TTA GTG GTG TCC AAA AAG CTC CGC AAC GGT CCA GAG GAA ACT ACC        2597
Ser Leu Val Val Ser Lys Lys Leu Arg Asn Gly Pro Glu Glu Thr Thr
            765                 770                 775

AAC GAG CAC CTC GTG GGT TAT TAT GTT TGT GAT AGC GCC TCA GTG TCC        2645
Asn Glu His Leu Val Gly Tyr Tyr Val Cys Asp Ser Ala Ser Val Ser
        780                 785                 790

GAG GCA GAC CTG CTG TCA TTT TTA GAG AAG AAA CTG CCT CGA TAC ATG        2693
Glu Ala Asp Leu Leu Ser Phe Leu Glu Lys Lys Leu Pro Arg Tyr Met
795                 800                 805                 810

ATT CCC ACG CGG TTG GTA CAG CTG TCG CAG ATC CCA GTG AAT GTG AAC        2741
Ile Pro Thr Arg Leu Val Gln Leu Ser Gln Ile Pro Val Asn Val Asn
            815                 820                 825
```

22

## Sequence Listing No. 1 (continued)

```
GGG AAG GCG GAC CTA CGC GCC TTG CCG GCC GTC GAT ATC TCC AAT TCC      2789
Gly Lys Ala Asp Leu Arg Ala Leu Pro Ala Val Asp Ile Ser Asn Ser
        830                 835                 840

ACG GAG GTG CGT TCC GAC CTT CGA GGC GAT ACG GAA ATC GCC CTC GGG      2837
Thr Glu Val Arg Ser Asp Leu Arg Gly Asp Thr Glu Ile Ala Leu Gly
        845                 850                 855

GAA ATC TGG GCC GAC GTG TTG GGA GCC CGC CAG AGA TCC GTC TCT CGC      2885
Glu Ile Trp Ala Asp Val Leu Gly Ala Arg Gln Arg Ser Val Ser Arg
        860                 865                 870

AAC GAC AAC TTC TTC CGC CTA GGA GGG CAC AGC ATC ACC TGC ATC CAA      2933
Asn Asp Asn Phe Phe Arg Leu Gly Gly His Ser Ile Thr Cys Ile Gln
875                 880                 885                 890

CTG ATC GCT CGC ATC CGA CAA CGA CAA CGA CTC TCG GTC AGC ATC TCC      2981
Leu Ile Ala Arg Ile Arg Gln Arg Gln Arg Leu Ser Val Ser Ile Ser
                895                 900                 905

GTC GAA GAT GTT TTT GCA ACA AGG ACA CTT GAG CGC ATG GCA GAC CTT      3029
Val Glu Asp Val Phe Ala Thr Arg Thr Leu Glu Arg Met Ala Asp Leu
                910                 915                 920

CTA CAG AAC AAG CAG CAG GAG AAA TGC GAC AAA CCC CAT GAG GCG CCG      3077
Leu Gln Asn Lys Gln Gln Glu Lys Cys Asp Lys Pro His Glu Ala Pro
        925                 930                 935

ACA GAG CTG CTT GAG GAG AAT GCA GCA ACG GAC AAT ATC TAT CTG GCA      3125
Thr Glu Leu Leu Glu Glu Asn Ala Ala Thr Asp Asn Ile Tyr Leu Ala
        940                 945                 950

AAC AGT CTT CAG CAG GGC TTC GTC TAC CAT TAC CTC AAG AGC ATG GAA      3173
Asn Ser Leu Gln Gln Gly Phe Val Tyr His Tyr Leu Lys Ser Met Glu
955                 960                 965                 970

CAA TCC GAC GCC TAT GTA ATG CAG TCC GTT CTT CGG TAC AAC ACC ACA      3221
Gln Ser Asp Ala Tyr Val Met Gln Ser Val Leu Arg Tyr Asn Thr Thr
                975                 980                 985

TTG TCT CCA GAT CTG TTT CAG AGA GCC TGG AAG CAT GCA CAG CAG TCC      3269
Leu Ser Pro Asp Leu Phe Gln Arg Ala Trp Lys His Ala Gln Gln Ser
                990                 995                 1000

TTT CCA GCG CTG CGG CTG CGG TTC TCA TGG GAA AAG GAG GTT TTC CAA      3317
Phe Pro Ala Leu Arg Leu Arg Phe Ser Trp Glu Lys Glu Val Phe Gln
        1005                1010                1015

CTG CTC GAT CAG GAT CCA CCA TTG GAC TGG CGT TTC CTC TAC TTC ACC      3365
Leu Leu Asp Gln Asp Pro Pro Leu Asp Trp Arg Phe Leu Tyr Phe Thr
        1020                1025                1030

GAC GTT GCC GCG GGT GCT GTC GAG GAC CGG AAA TTG GAA GAC TTG CGG      3413
Asp Val Ala Ala Gly Ala Val Glu Asp Arg Lys Leu Glu Asp Leu Arg
1035                1040                1045                1050
```

23

**Sequence Listing No. 1 (continued)**

```
CGC CAA GAC CTT ACG GAG AGA TTC AAG CTG GAT GTT GGC AGA CTG TTC      3461
Arg Gln Asp Leu Thr Glu Arg Phe Lys Leu Asp Val Gly Arg Leu Phe
        1055              1060              1065

CGC GTC TAT CTG ATT AAA CAC AGC GAG AAT CGC TTC ACG TGT CTT TTC      3509
Arg Val Tyr Leu Ile Lys His Ser Glu Asn Arg Phe Thr Cys Leu Phe
        1070              1075              1080

AGC TGC CAG CAT GCA ATC CTC GAT GGT TGG AGT CTG CCA CTC TTG TTC      3557
Ser Cys Gln His Ala Ile Leu Asp Gly Trp Ser Leu Pro Leu Leu Phe
        1085              1090              1095

GAA AAG GTT CAC GAG ACC TAC CTG CAA CTG CTG CAT GGG GAC AAT CTC      3605
Glu Lys Val His Glu Thr Tyr Leu Gln Leu Leu His Gly Asp Asn Leu
        1100              1105              1110

ACT TCG TCC ATG GAT GAC CCT TAC ACT CGC ACC CAG CGG TAT CTC CAC      3653
Thr Ser Ser Met Asp Asp Pro Tyr Thr Arg Thr Gln Arg Tyr Leu His
    1115              1120              1125              1130

GCT CAC CGT GAG GAT CAC CTC GAC TTT TGG GCC GGT GTG GTT CAA AAG      3701
Ala His Arg Glu Asp His Leu Asp Phe Trp Ala Gly Val Val Gln Lys
        1135              1140              1145

ATC AAC GAA CGG TGT GAT ATG AAC GCC TTG TTG AAC GAG CGC AGT CGT      3749
Ile Asn Glu Arg Cys Asp Met Asn Ala Leu Leu Asn Glu Arg Ser Arg
        1150              1155              1160

TAC AAA GTC CAG CTG GCA GAC TAT GAC CAG GTG CAG GAG CAG CGA CAC      3797
Tyr Lys Val Gln Leu Ala Asp Tyr Asp Gln Val Gln Glu Gln Arg His
        1165              1170              1175

GTG ACA ATT GCT CTC TCT GGA GAC GCA TGG CTA GCA GAC CTT CGT CAG      3845
Val Thr Ile Ala Leu Ser Gly Asp Ala Trp Leu Ala Asp Leu Arg Gln
        1180              1185              1190

ACC TGC TCC GCC CAG GGT ATT ACC TTA CAT TCG ATT CTC CAA TTT GTT      3893
Thr Cys Ser Ala Gln Gly Ile Thr Leu His Ser Ile Leu Gln Phe Val
    1195              1200              1205              1210

TGG CAC GCC GTG CTG CAC GCT TAT GGC GGT GGC ACC CAC ACC ATA ACC      3941
Trp His Ala Val Leu His Ala Tyr Gly Gly Gly Thr His Thr Ile Thr
        1215              1220              1225

GGC ACG ACC ATT TCT GGA AGG AAC CTG CCC ATC TTG GGA ATT GAA CGA      3989
Gly Thr Thr Ile Ser Gly Arg Asn Leu Pro Ile Leu Gly Ile Glu Arg
        1230              1235              1240

GCA GTT GGT CCG TAT ATC AAC ACT CTA CCG CTG GTA CTC GAT CAT TCG      4037
Ala Val Gly Pro Tyr Ile Asn Thr Leu Pro Leu Val Leu Asp His Ser
        1245              1250              1255

ACG TTC AAG GAT AAG ACA ATC ATG GAG GCC ATC GAG GAT GTG CAG GCC      4085
Thr Phe Lys Asp Lys Thr Ile Met Glu Ala Ile Glu Asp Val Gln Ala
        1260              1265              1270
```

24

## Sequence Listing No. 1 (continued)

AAG GTA AAC GTC ATG AAC AGC CGG GGC AAT GTG GAA CTG GGC CGT TTG     4133
Lys Val Asn Val Met Asn Ser Arg Gly Asn Val Glu Leu Gly Arg Leu
1275            1280            1285           1290

CAC AAA ACC GAC TTA AAG CAC GGA TTA TTC GAT TCT TTA TTC GTG CTT     4181
His Lys Thr Asp Leu Lys His Gly Leu Phe Asp Ser Leu Phe Val Leu
            1295           1300           1305

GAA AAC TAC CCG AAT TTG GAC AAA TCG CGA ACA CTT GAG CAC CAG ACT     4229
Glu Asn Tyr Pro Asn Leu Asp Lys Ser Arg Thr Leu Glu His Gln Thr
        ·1310           1315           1320

GAA CTG GGG TAT TCG ATT GAA GGC GGC ACT GAG AAG CTG AAT TAT CCA     4277
Glu Leu Gly Tyr Ser Ile Glu Gly Gly Thr Glu Lys Leu Asn Tyr Pro
       1325           1330           1335

CTG GCT GTC ATC GCG CGC GAA GTC GAG ACG ACT GGC GGA TTC ACA GTA     4325
Leu Ala Val Ile Ala Arg Glu Val Glu Thr Thr Gly Gly Phe Thr Val
      1340           1345           1350

TCC ATC TGC TAC GCC AGT GAG CTA TTT GAG GAG GTT ATG ATC TCC GAG     4373
Ser Ile Cys Tyr Ala Ser Glu Leu Phe Glu Glu Val Met Ile Ser Glu
1355            1360           1365           1370

CTT CTT CAT ATG GTC CAG GAC ACA CTG ATG CAG GTT GCC CGA GGT TTG     4421
Leu Leu His Met Val Gln Asp Thr Leu Met Gln Val Ala Arg Gly Leu
            1375           1380           1385

AAT GAA CCC GTC GGC AGC CTG GAG TAT CTC TCA TCT ATC CAA TTG GAG     4469
Asn Glu Pro Val Gly Ser Leu Glu Tyr Leu Ser Ser Ile Gln Leu Glu
            1390           1395           1400

CAA CTC GCC GCG TGG AAT GCC ACG GAA GCT GAG TTT CCC GAT ACC ACG     4517
Gln Leu Ala Ala Trp Asn Ala Thr Glu Ala Glu Phe Pro Asp Thr Thr
        1405           1410           1415

CTT CAT GAG ATG TTT GAA AAC GAA GCG AGC CAG AAG CCG GAC AAG ATA     4565
Leu His Glu Met Phe Glu Asn Glu Ala Ser Gln Lys Pro Asp Lys Ile
       1420           1425           1430

GCA GTG GTC TAT GAG GAG ACG TCC TTG ACT TAC CGC GAG TTG AAT GAG     4613
Ala Val Val Tyr Glu Glu Thr Ser Leu Thr Tyr Arg Glu Leu Asn Glu
1435            1140           1445           1450

CGG GCG AAC CGT ATG GCA CAT CAG CTA AGG TCC GAC GTC AGC CCC AAC     4661
Arg Ala Asn Arg Met Ala His Gln Leu Arg Ser Asp Val Ser Pro Asn
            1455           1460           1465

CCC AAC GAG GTC ATT GCG CTG GTG ATG GAC AAG AGC GAG CAT ATG ATC     4709
Pro Asn Glu Val Ile Ala Leu Val Met Asp Lys Ser Glu His Met Ile
            1470           1475           1480

GTC AAC ATT CTG GCC GTA TGG AAG AGC GGC GGT GCC TAT GTC CCC ATT     4757
Val Asn Ile Leu Ala Val Trp Lys Ser Gly Gly Ala Tyr Val Pro Ile
       1485           1490           1495

**Sequence Listing No. 1 (continued)**

```
GAC CCT GGA TAT CCT AAC GAC CGC ATT CAA TAT ATC CTA GAG GAC ACA      4805
Asp Pro Gly Tyr Pro Asn Asp Arg Ile Gln Tyr Ile Leu Glu Asp Thr
    1500                1505                1510

CAA GCC CTC GCA GTC ATC GCG GAC TCC TGC TAT CTG CCT CGC ATC AAG      4853
Gln Ala Leu Ala Val Ile Ala Asp Ser Cys Tyr Leu Pro Arg Ile Lys
1515                1520                1525                1530

GGA ATG GCT GCC TCC GGC ACG CTT CTT TAT CCC TCT GTC TTG CCT GCC      4901
Gly Met Ala Ala Ser Gly Thr Leu Leu Tyr Pro Ser Val Leu Pro Ala
                1535                1540                1545

AAT CCG GAT TCC AAG TGG AGC GTA TCG AAC CCT TCA CCG TTG AGT CGG      4949
Asn Pro Asp Ser Lys Trp Ser Val Ser Asn Pro Ser Pro Leu Ser Arg
                1550                1555                1560

AGC ACG GAC TTA GCT TAT ATC ATC TAT ACC TCT GGA ACG ACA GGT CGG      4997
Ser Thr Asp Leu Ala Tyr Ile Ile Tyr Thr Ser Gly Thr Thr Gly Arg
            1565                1570                1575

CCC AAG GGC GTC ACG GTA GAG CAT CAT GGA GTG GTC AAC CTG CAG GTG      5045
Pro Lys Gly Val Thr Val Glu His His Gly Val Val Asn Leu Gln Val
        1580                1585                1590

TCG CTA TCC AAA GTA TTC GGA CTA CGG GAT ACG GAC GAC GAG GTA ATT      5093
Ser Leu Ser Lys Val Phe Gly Leu Arg Asp Thr Asp Asp Glu Val Ile
    1595                1600                1605                1610

CTC TCC TTT TCC AAC TAT GTG TTC GAC CAT TTC GTG GAG CAG ATG ACC      5141
Leu Ser Phe Ser Asn Tyr Val Phe Asp His Phe Val Glu Gln Met Thr
                1615                1620                1625

GAC GCC ATT CTC AAT GGC CAA ACC CTC CTG GTC CTC AAC GAT GGA ATG      5189
Asp Ala Ile Leu Asn Gly Gln Thr Leu Leu Val Leu Asn Asp Gly Met
            1630                1635                1640

CGC GGG GAC AAA GAG CGA CTC TAC AGA TAC ATT GAG AAG AAC CGA GTG      5237
Arg Gly Asp Lys Glu Arg Leu Tyr Arg Tyr Ile Glu Lys Asn Arg Val
        1645                1650                1655

ACC TAC TTG TCT GGC ACC CCA TCC GTG GTC TCC ATG TAC GAA TTT AGC      5285
Thr Tyr Leu Ser Gly Thr Pro Ser Val Val Ser Met Tyr Glu Phe Ser
        1660                1665                1670

CGG TTC AAG GAC CAT CTA CGC CGT GTG GAC TGC GTG GGG GAG GCG TTC      5333
Arg Phe Lys Asp His Leu Arg Arg Val Asp Cys Val Gly Glu Ala Phe
1675                1680                1685                1690

AGC GAA CCG GTC TTC GAC AAG ATC CGC GAA ACG TTC CAT GGC CTC GTT      5381
Ser Glu Pro Val Phe Asp Lys Ile Arg Glu Thr Phe His Gly Leu Val
            1695                1700                1705

ATC AAC GGC TAC GGC CCA ACT GAA GTT TCC ATC ACC ACC CAC AAG CGG      5429
Ile Asn Gly Tyr Gly Pro Thr Glu Val Ser Ile Thr Thr His Lys Arg
            1710                1715                1720
```

26

## Sequence Listing No. 1 (continued)

```
CTC TAT CCA TTC CCA GAG CGG CGA ATG GAC AAA AGT ATT GGC CAA CAG      5477
Leu Tyr Pro Phe Pro Glu Arg Arg Met Asp Lys Ser Ile Gly Gln Gln
        1725                1730                1735

GTC CAC AAT AGC ACG AGC TAT GTG CTG AAC GAG GAC ATG AAG CGC ACC      5525
Val His Asn Ser Thr Ser Tyr Val Leu Asn Glu Asp Met Lys Arg Thr
        1740                1745                1750

CCC ATA GGG GCT GTC GGC GAG CTC TAC CTG GGT GGT GAA GGA GTG GTA      5573
Pro Ile Gly Ala Val Gly Glu Leu Tyr Leu Gly Gly Glu Gly Val Val
1755                1760                1765                1770

CGG GGA TAT CAC AAT CGC GCA GAT GTG ACC GCG GAG CGT TTT ATT CCT      5621
Arg Gly Tyr His Asn Arg Ala Asp Val Thr Ala Glu Arg Phe Ile Pro
                1775                1780                1785

AAT CCA TTC CAG TCG GAA GAA GAT AAG CGA GAA GGT CGT AAC TCC CGT      5669
Asn Pro Phe Gln Ser Glu Glu Asp Lys Arg Glu Gly Arg Asn Ser Arg
                1790                1795                1800

TTG TAC AAG ACC GGT GAC CTG GTA CGC TGG ATT CCT GGA AGC AGC GGG      5717
Leu Tyr Lys Thr Gly Asp Leu Val Arg Trp Ile Pro Gly Ser Ser Gly
        1805                1810                1815

GAG GTC GAG TAT CTA GGT CGT AAT GAC TTC CAG GTC AAG ATT CGC GGA      5765
Glu Val Glu Tyr Leu Gly Arg Asn Asp Phe Gln Val Lys Ile Arg Gly
        1820                1825                1830

CTG CGC ATC GAA GTA GGC GAG ATT GAG GCC ATC CTA TCG TCT TAT CAC      5813
Leu Arg Ile Glu Val Gly Glu Ile Glu Ala Ile Leu Ser Ser Tyr His
1835                1840                1845                1850

GGA ATC AAA CAG TCT GTG GTG ATT GCC AAG GAT TGC AGA GAA GGG GCC      5861
Gly Ile Lys Gln Ser Val Val Ile Ala Lys Asp Cys Arg Glu Gly Ala
                1855                1860                1865

CAG AAA TTC CTG GTT GGT TAC TAT GTC GCC GAT GCA GCG CTG CCG TCC      5909
Gln Lys Phe Leu Val Gly Tyr Tyr Val Ala Asp Ala Ala Leu Pro Ser
                1870                1875                1880

GCT GCC ATT CGG CGC TTC ATG CAG TCT CGG CTC CCT GGC TAC ATG GTG      5957
Ala Ala Ile Arg Arg Phe Met Gln Ser Arg Leu Pro Gly Tyr Met Val
                1885                1890                1895

CCC TCT CGT CTC ATT CTC GTC AGC AAG TTC CCC GTC ACT CCT AGT GGA      6005
Pro Ser Arg Leu Ile Leu Val Ser Lys Phe Pro Val Thr Pro Ser Gly
        1900                1905                1910

AAA TTA GAC ACC AAG GCT TTG CCC CCA GCC GAG GAA GAG AGC GAG ATT      6053
Lys Leu Asp Thr Lys Ala Leu Pro Pro Ala Glu Glu Glu Ser Glu Ile
1915                1920                1925                1930

GAC GTG GTG CCG CCG CGT AGT GAA ATC GAA CGC TCC TTG TGT GAC ATC      6101
Asp Val Val Pro Pro Arg Ser Glu Ile Glu Arg Ser Leu Cys Asp Ile
                1935                1940                1945
```

27

**Sequence Listing No. 1 (continued)**

```
TGG GCG GAA CTA CTC GAG ATG CAC CCA GAG GAG ATC GGC ATT TAC AGC          6149
Trp Ala Glu Leu Leu Glu Met His Pro Glu Glu Ile Gly Ile Tyr Ser
          1950                    1955                1960

GAT TTC TTC AGC CTG GGA GGT GAC AGC CTA AAG AGC ACA AAG CTT TCC          6197
Asp Phe Phe Ser Leu Gly Gly Asp Ser Leu Lys Ser Thr Lys Leu Ser
          1965                    1970                1975

TTC ATG ATT CAC GAG TCC TTT AAC CGC GCC GTC TCA GTC AGC GCC CTT          6245
Phe Met Ile His Glu Ser Phe Asn Arg Ala Val Ser Val Ser Ala Leu
          1980                    1985                1990

TTC TGT CAC CGG ACA GTT GAA GCC CAG ACG CAC TTG ATC CTG AAC GAT          6293
Phe Cys His Arg Thr Val Glu Ala Gln Thr His Leu Ile Leu Asn Asp
1995                    2000                2005                2010

GCT GCA GAT GTG CAC GAA ATT ACT CCC ATA GAT TGC AAT GAT ACG CAG          6341
Ala Ala Asp Val His Glu Ile Thr Pro Ile Asp Cys Asn Asp Thr Gln
          2015                    2020                2025

ATG ATT CCC GTG TCC CGT GCC CAG GAG CGA CTC CTC TTC ATC CAC GAA          6389
Met Ile Pro Val Ser Arg Ala Gln Glu Arg Leu Leu Phe Ile His Glu
          2030                    2035                2040

TTT GAG AAT GGC AGC AAT GCA TAC AAT ATC GAC GCT GCA TTT GAA CTG          6437
Phe Glu Asn Gly Ser Asn Ala Tyr Asn Ile Asp Ala Ala Phe Glu Leu
          2045                    2050                2055

CCT GGC TCG GTT GAC GCG TCG CTT CTC GAG CAG GCG CTG CGT GGA AAC          6485
Pro Gly Ser Val Asp Ala Ser Leu Leu Glu Gln Ala Leu Arg Gly Asn
          2060                    2065                2070

CTT GCT CGA CAT GAG GCG TTG AGA ACT TTA CTG GTC AAG GAT CAC GCA          6533
Leu Ala Arg His Glu Ala Leu Arg Thr Leu Leu Val Lys Asp His Ala
2075                    2080                2085                2090

ACC GGC ATC TAT CTT CAG AAG GTA TTG AGT CCC GAT GAA GCC CAG GGC          6581
Thr Gly Ile Tyr Leu Gln Lys Val Leu Ser Pro Asp Glu Ala Gln Gly
          2095                    2100                2105

ATG TTC TCC GTC AAC GTG GAC ACA GCC AAG CAG GTG GAG CGG CTG GAC          6629
Met Phe Ser Val Asn Val Asp Thr Ala Lys Gln Val Glu Arg Leu Asp
          2110                    2115                2120

CAG GAG ATA GCC AGT CTA TCC CAG CAT GTT TTC CGC CTC GAT GAT GAA          6677
Gln Glu Ile Ala Ser Leu Ser Gln His Val Phe Arg Leu Asp Asp Glu
          2125                    2130                2135

CTG CCT TGG GAG GCC CGC ATC CTT AAA CTC GAA TCC GGC GGC CTG TAT          6725
Leu Pro Trp Glu Ala Arg Ile Leu Lys Leu Glu Ser Gly Gly Leu Tyr
          2140                    2145                2150

CTC ATT CTG GCG TTC CAC CAT ACC TGC TTC GAT GCA TGG TCA TTG AAA          6773
Leu Ile Leu Ala Phe His His Thr Cys Phe Asp Ala Trp Ser Leu Lys
2155                    2160                2165                2170
```

## Sequence Listing No. 1 (continued)

```
GTC TTC GAG CAA GAG CTT CGG GCC TTG TAC GCA GCG CTC CAG AAA ACC          6821
Val Phe Glu Gln Glu Leu Arg Ala Leu Tyr Ala Ala Leu Gln Lys Thr
            2175                    2180                2185

AAA AGT GCA GCG AAC TTA CCA GCC CTC AAA GCG CAG TAC AAG GAA TAC          6869
Lys Ser Ala Ala Asn Leu Pro Ala Leu Lys Ala Gln Tyr Lys Glu Tyr
            2190                    2195                2200

GCG CTC TAC CAT CGC CGG CAG CTG TCT GGC GAT CGC ATG CGC AAC CTG          6917
Ala Leu Tyr His Arg Arg Gln Leu Ser Gly Asp Arg Met Arg Asn Leu
            2205                    2210                2215

TCA GAC TTT TGG CTG CGG AAA CTC ATT GGC TTG GAA CCA TTG CAG CTG          6965
Ser Asp Phe Trp Leu Arg Lys Leu Ile Gly Leu Glu Pro Leu Gln Leu
            2220                    2225                2230

ATC ACG GAC CGC CCA CGT CCT GTG CAA TTC AAA TAC GAC GGT GAC GAC          7013
Ile Thr Asp Arg Pro Arg Pro Val Gln Phe Lys Tyr Asp Gly Asp Asp
2235                    2240                    2245                2250

CTC AGT ATC GAA CTG AGC AAG AAG GAA ACG GAG AAC CTG AGG GGG GTG          7061
Leu Ser Ile Glu Leu Ser Lys Lys Glu Thr Glu Asn Leu Arg Gly Val
            2255                    2260                2265

GCC AAA CGT TGC AAG TCG AGT CTG TAC GTC GTG TTG GTT TCC GTT TAT          7109
Ala Lys Arg Cys Lys Ser Ser Leu Tyr Val Val Leu Val Ser Val Tyr
            2270                    2275                2280

TGC GTT ATG CTA GCC TCG TAC GCG AAC CAG TCC GAT GTT TCC GTG GGT          7157
Cys Val Met Leu Ala Ser Tyr Ala Asn Gln Ser Asp Val Ser Val Gly
            2285                    2290                2295

ATC CCA GTC AGC CAC CGA ACG CAT CCT CAG TTC CAA TCG GTC ATT GGA          7205
Ile Pro Val Ser His Arg Thr His Pro Gln Phe Gln Ser Val Ile Gly
            2300                    2305                2310

TTC TTC GTC AAC CTT GTG GTG CTA AGG GTG GAT ATT TCT CAG TCA GCC          7253
Phe Phe Val Asn Leu Val Val Leu Arg Val Asp Ile Ser Gln Ser Ala
2315                    2320                    2325                2330

ATT TGC GGG CTC ATC AGA AGG GTA ATG AAA GAG CTC GTG GAC GCC CAA          7301
Ile Cys Gly Leu Ile Arg Arg Val Met Lys Glu Leu Val Asp Ala Gln
            2335                    2340                2345

CTG CAC CAA GAC ATG CCG TTC CAG GAA GTG ACG AAG CTG CTG CAG GTG          7349
Leu His Gln Asp Met Pro Phe Gln Glu Val Thr Lys Leu Leu Gln Val
            2350                    2355                2360

GAT AAT GAC CCC AGC CGG CAT CCG CTG GTA CAG AAC GTG TTC AAC TTC          7397
Asp Asn Asp Pro Ser Arg His Pro Leu Val Gln Asn Val Phe Asn Phe
            2365                    2370                2375

GAA TCC CGT GCG AAC GGA GAA CAC GAT GCC AGG TCG GAG GAT GAA GGA          7445
Glu Ser Arg Ala Asn Gly Glu His Asp Ala Arg Ser Glu Asp Glu Gly
            2380                    2385                2390
```

**Sequence Listing No. 1 (continued)**

```
TCG CTT GCA TTC AAT CAA TAC CGG CCG GTT CAG CCC GTG GAT TCC GTT      7493
Ser Leu Ala Phe Asn Gln Tyr Arg Pro Val Gln Pro Val Asp Ser Val
2395                2400                2405                2410

GCG AAG TTC GAT CTG AAC GCA ACG GTC ACG GAA TTG GAG TCG GGA TTG      7541
Ala Lys Phe Asp Leu Asn Ala Thr Val Thr Glu Leu Glu Ser Gly Leu
                2415                2420                2425

AGA GTC AAC TTC AAC TAT GCG ACC AGC CTA TTC AAC AAA AGC ACG ATC      7589
Arg Val Asn Phe Asn Tyr Ala Thr Ser Leu Phe Asn Lys Ser Thr Ile
                2430                2435                2440

CAG GGT TTT TTG CAT ACC TAT GAG TAT CTC CTG CGC CAG CTG TCC GAA      7637
Gln Gly Phe Leu His Thr Tyr Glu Tyr Leu Leu Arg Gln Leu Ser Glu
                2445                2450                2455

CTG AGT GCA GAA GGG ATC AAT GAG GAT ACG CAG CTG TCG TTA GTT CGC      7685
Leu Ser Ala Glu Gly Ile Asn Glu Asp Thr Gln Leu Ser Leu Val Arg
     2460                2465                2470

CCG ACA GAG AAT GGC GAT CTG CAC TTG CCA TTG GCA CAG TCC CCG CTT      7733
Pro Thr Glu Asn Gly Asp Leu His Leu Pro Leu Ala Gln Ser Pro Leu
2475                2480                2485                2490

GCG ACG ACT GCT GAG GAG CAG AAA GTA GCG TCG TTG AAC CAG GCC TTT      7781
Ala Thr Thr Ala Glu Glu Gln Lys Val Ala Ser Leu Asn Gln Ala Phe
                2495                2500                2505

GAG CGC GAA GCT TTC CTT GCC GCA GAG AAG ATT GCC GTC GTG CAG GGA      7829
Glu Arg Glu Ala Phe Leu Ala Ala Glu Lys Ile Ala Val Val Gln Gly
                2510                2515                2520

GAT AGA GCA CTT AGT TAT GCT GAT CTT AAC GGG CAG GCT AAC CAG CTC      7877
Asp Arg Ala Leu Ser Tyr Ala Asp Leu Asn Gly Gln Ala Asn Gln Leu
                2525                2530                2535

GCC CGG TAC ATA CAG TCC GTG TCC TGT ATT GGG GCA GAC GAC GGA ATA      7925
Ala Arg Tyr Ile Gln Ser Val Ser Cys Ile Gly Ala Asp Asp Gly Ile
     2540                2545                2550

GCT TTG ATG CTG GAA AAG AGT ATC GAC ACG ATT ATT TGC ATT CTC GCG      7973
Ala Leu Met Leu Glu Lys Ser Ile Asp Thr Ile Ile Cys Ile Leu Ala
2555                2560                2565                2570

ATT TGG AAG GCT GGT GCA GCA TAC GTG CCC TTG GAT CCG ACT TAC CCA      8021
Ile Trp Lys Ala Gly Ala Ala Tyr Val Pro Leu Asp Pro Thr Tyr Pro
                2575                2580                2585

CCC GGA CGC GTC CAG CTG ATT CTG GAG GAG ATT AAA GCG AAG GCT GTC      8069
Pro Gly Arg Val Gln Leu Ile Leu Glu Glu Ile Lys Ala Lys Ala Val
                2590                2595                2600

CTT GTG CAC TCC AGT CAT GCT TCG AAA TGT GAA CGC CAT GGC GCG AAG      8117
Leu Val His Ser Ser His Ala Ser Lys Cys Glu Arg His Gly Ala Lys
     2605                2610                2615
```

## Sequence Listing No. 1 (continued)

```
GTG ATT GCA GTC GAC TCG CCC GCC ATC GAG ACG GCG GTC AGC CAA CAG        8165
Val Ile Ala Val Asp Ser Pro Ala Ile Glu Thr Ala Val Ser Gln Gln
    2620                2625                2630

TCA GCT GCT GAC CTG CCC ACA ATT GCT AGC CTC GGC AAT CTA GCG TAT        8213
Ser Ala Ala Asp Leu Pro Thr Ile Ala Ser Leu Gly Asn Leu Ala Tyr
2635                2640                2645                2650

ATA ATC TTT ACT TCA GGC ACT TCC GGT AAG CCA AAG GGA GTC CTA GTT        8261
Ile Ile Phe Thr Ser Gly Thr Ser Gly Lys Pro Lys Gly Val Leu Val
                2655                2660                2665

GAG CAA AAG GCA GTT CTT CTT CTA CGC GAT GCC CTC CGG GAG CGG TAT        8309
Glu Gln Lys Ala Val Leu Leu Leu Arg Asp Ala Leu Arg Glu Arg Tyr
            2670                2675                2680

TTC GGT CGA GAC TGT ACC AAG CAT CAT GGC GTC CTG TTC CTG TCC AAC        8357
Phe Gly Arg Asp Cys Thr Lys His His Gly Val Leu Phe Leu Ser Asn
        2685                2690                2695

TAC GTC TTC GAC TTC TCC GTC GAA CAA CTT GTG TTG TCG GTG CTC AGC        8405
Tyr Val Phe Asp Phe Ser Val Glu Gln Leu Val Leu Ser Val Leu Ser
    2700                2705                2710

GGA CAC AAG CTG ATC GTT CCC CCA GCT GAG TTC GTC GCA GAT GAT GAA        8453
Gly His Lys Leu Ile Val Pro Pro Ala Glu Phe Val Ala Asp Asp Glu
2715                2720                2725                2730

TTT TAC AGA ATG GCC AGC ACG CAC GGT CTC TCC TAT CTC AGC GGC ACA        8501
Phe Tyr Arg Met Ala Ser Thr His Gly Leu Ser Tyr Leu Ser Gly Thr
                2735                2740                2745

CCA TCC TTA CTG CAG AAG ATC GAT CTG GCA CGA CTG GAC CAT CTG CAG        8549
Pro Ser Leu Leu Gln Lys Ile Asp Leu Ala Arg Leu Asp His Leu Gln
                2750                2755                2760

GTT GTT ACC GCC GCG GGC GAA GAG CTT CAC GCC ACC CAG TAC GAG AAG        8597
Val Val Thr Ala Ala Gly Glu Glu Leu His Ala Thr Gln Tyr Glu Lys
            2765                2770                2775

ATG CGC CGC CGA TTC AAC GGT CCC ATC TAC AAT GCC TAT GGT GTC ACC        8645
Met Arg Arg Arg Phe Asn Gly Pro Ile Tyr Asn Ala Tyr Gly Val Thr
        2780                2785                2790

GAG ACC ACG GTG TAC AAC ATT ATC GCG GAA TTC ACA ACG AAT TCG ATA        8693
Glu Thr Thr Val Tyr Asn Ile Ile Ala Glu Phe Thr Thr Asn Ser Ile
2795                2800                2805                2810

TTT GAG AAT GCT CTT CGG GAA GTG CTC CCT GGT ACC CGA GCG TAT GTG        8741
Phe Glu Asn Ala Leu Arg Glu Val Leu Pro Gly Thr Arg Ala Tyr Val
                2815                2820                2825

CTG AAC GCG GCA CTT CAG CCC GTC CCC TTC GAT GCT GTC GGA GAA CTC        8789
Leu Asn Ala Ala Leu Gln Pro Val Pro Phe Asp Ala Val Gly Glu Leu
                2830                2835                2840
```

**Sequence Listing No. 1 (continued)**

```
TAT CTT GCC GGC GAC ACG GTT ACG CGT GGT TAT CTC AAC CAA CCT CTT       8837
Tyr Leu Ala Gly Asp Thr Val Thr Arg Gly Tyr Leu Asn Gln Pro Leu
        2845                2850                2855

CTA ACG GAT CAG CGA TTC ATT CCC AAC CCT TTC TGC AAA GAG GAG GAC       8885
Leu Thr Asp Gln Arg Phe Ile Pro Asn Pro Phe Cys Lys Glu Glu Asp
        2860                2865                2870

ATC GCT ATG GGG CGC TTC GCG CGG CTC TAC AAG ACC GGC GAC CTG GTT       8933
Ile Ala Met Gly Arg Phe Ala Arg Leu Tyr Lys Thr Gly Asp Leu Val
  2875                2880                2885                2890

CGA TCG CGT TTC AAC CGT CAG CAG CAG CCG CAG CTG GAA TAC CTA GGA       8981
Arg Ser Arg Phe Asn Arg Gln Gln Gln Pro Gln Leu Glu Tyr Leu Gly
                2895                2900                2905

AGA GGC GAT CTG CAG ATC AAG ATG AGG GGA TAC CGG ATC GAG ATT TCT       9029
Arg Gly Asp Leu Gln Ile Lys Met Arg Gly Tyr Arg Ile Glu Ile Ser
                2910                2915                2920

GAA GTT CAG AAC GTG CTC ACT TCA AGT CCC GGT GTC CGG GAG GGT GCA       9077
Glu Val Gln Asn Val Leu Thr Ser Ser Pro Gly Val Arg Glu Gly Ala
            2925                2930                2935

GTC GTT GCC AAG TAT GAG AAC AAC GAT ACC TAT TCC CGG ACC GCT CAC       9125
Val Val Ala Lys Tyr Glu Asn Asn Asp Thr Tyr Ser Arg Thr Ala His
        2940                2945                2950

TCT CTG GTC GGT TAC TAT ACC ACG GAC AAT GAA ACA GTA TCG GAA GCC       9173
Ser Leu Val Gly Tyr Tyr Thr Thr Asp Asn Glu Thr Val Ser Glu Ala
  2955                2960                2965                2970

GAT ATT CTC ACT TTC ATG AAA GCA AGG CTT CCA ACG TAC ATG GTG CCA       9221
Asp Ile Leu Thr Phe Met Lys Ala Arg Leu Pro Thr Tyr Met Val Pro
                2975                2980                2985

AGC CAC CTC TGC TGT CTG GAA GGC GCA CTG CCT GTG ACG ATT AAC GGA       9269
Ser His Leu Cys Cys Leu Glu Gly Ala Leu Pro Val Thr Ile Asn Gly
                2990                2995                3000

AAG CTC GAC GTC CGG AGA TTG CCG GAG ATT ATC AAC GAC TCC GCG CAG       9317
Lys Leu Asp Val Arg Arg Leu Pro Glu Ile Ile Asn Asp Ser Ala Gln
        3005                3010                3015

TCC TCG TAC AGC CCA CCA AGG AAC ATA ATC GAG GCC AAG ATG TGC AGA       9365
Ser Ser Tyr Ser Pro Pro Arg Asn Ile Ile Glu Ala Lys Met Cys Arg
        3020                3025                3030

CTG TGG GAA TCC GCC TTG GGA ATG GAG CGA TGC GGT ATC GAC GAC GAC       9413
Leu Trp Glu Ser Ala Leu Gly Met Glu Arg Cys Gly Ile Asp Asp Asp
  3035                3040                3045                3050

CTG TTC AAA CTG GGT GGC GAC AGC ATC ACA TCT TTG CAT CTC GTG GCC       9461
Leu Phe Lys Leu Gly Gly Asp Ser Ile Thr Ser Leu His Leu Val Ala
                3055                3060                3065
```

## Sequence Listing No. 1 (continued)

```
CAG ATT CAC AAC CAG GTG GGC TGC AAG ATC ACC GTT CGG GAT ATA TTT    9509
Gln Ile His Asn Gln Val Gly Cys Lys Ile Thr Val Arg Asp Ile Phe
         3070                3075                3080

GAA CAT CGT ACC GCC CGA GCC CTC CAT GAT CAC GTC TTC ATG AAG GAC    9557
Glu His Arg Thr Ala Arg Ala Leu His Asp His Val Phe Met Lys Asp
         3085                3090                3095

TCC GAC CGG AGT AAT GTG ACT CAG TTC CGA ACC GAA CAA GGG CCG GTC    9605
Ser Asp Arg Ser Asn Val Thr Gln Phe Arg Thr Glu Gln Gly Pro Val
         3100                3105                3110

ATC GGC GAG GCG CCC CTA CTG CCG ATT CAA GAC TGG TTT TTG TCA AAG    9653
Ile Gly Glu Ala Pro Leu Leu Pro Ile Gln Asp Trp Phe Leu Ser Lys
3115                3120                3125                3130

GCT CTG CAG CAT CCG ATG TAT TGG AAT CAC ACT TTC TAC GTC CGA ACG    9701
Ala Leu Gln His Pro Met Tyr Trp Asn His Thr Phe Tyr Val Arg Thr
              3135                3140                3145

CCA GAG CTG GAT GTT GAT TCC TTA AGC GCT GCT GTC AGG GAC TTG CAA    9749
Pro Glu Leu Asp Val Asp Ser Leu Ser Ala Ala Val Arg Asp Leu Gln
              3150                3155                3160

CAG TAT CAC GAT GTT TTC CGC ATG CGA CTC AAG CGC GAG GAA GTC GGA    9797
Gln Tyr His Asp Val Phe Arg Met Arg Leu Lys Arg Glu Glu Val Gly
         3165                3170                3175

TTC GTG CAG TCC TTT GCT GAG GAC TTC TCT CCT GCC CAG CTT CGG GTG    9845
Phe Val Gln Ser Phe Ala Glu Asp Phe Ser Pro Ala Gln Leu Arg Val
         3180                3185                3190

CTG AAC GTA AAA GAT GTT GAC GGG TCC GCG GCC GTC AAC GAG ATA TTG    9893
Leu Asn Val Lys Asp Val Asp Gly Ser Ala Ala Val Asn Glu Ile Leu
3195                3200                3205                3210

GAT GGG TGG CAG TCT GGC TTC AAC CTT GAG AAC GGA CCC ATT GGT TCC    9941
Asp Gly Trp Gln Ser Gly Phe Asn Leu Glu Asn Gly Pro Ile Gly Ser
              3215                3220                3225

ATT GGC TAC CTA CAT GGG TAT GAA GAC CGA TCC GCG CGA GTC TGG TTC    9989
Ile Gly Tyr Leu His Gly Tyr Glu Asp Arg Ser Ala Arg Val Trp Phe
              3230                3235                3240

TCC GTT CAC CAT ATG GCC ATT GAC ACC GTC AGC TGG CAG ATC CTT GTC    10037
Ser Val His His Met Ala Ile Asp Thr Val Ser Trp Gln Ile Leu Val
         3245                3250                3255

CGT GAC CTG CAG ACG CTG TAC CGA AAT GGA AGC CTC GGA AGC AAG GGC    10085
Arg Asp Leu Gln Thr Leu Tyr Arg Asn Gly Ser Leu Gly Ser Lys Gly
         3260                3265                3270

AGC AGT TTC CGG CAG TGG GCT GAA GCC ATC CAA AAT TAC AAG GCG TCA    10133
Ser Ser Phe Arg Gln Trp Ala Glu Ala Ile Gln Asn Tyr Lys Ala Ser
3275                3280                3285                3290
```

**Sequence Listing No. 1 (continued)**

```
GAC TCT GAG AGG AAC CAT TGG AAT AAG CTC GTC ATG GAA ACA GCT TCC    10181
Asp Ser Glu Arg Asn His Trp Asn Lys Leu Val Met Glu Thr Ala Ser
              3295                3300                3305

AGC ATA TCC GCA TTG CCT ACG TCA ACC GGT TCG CGC GTG CGC CTG AGC    10229
Ser Ile Ser Ala Leu Pro Thr Ser Thr Gly Ser Arg Val Arg Leu Ser
              3310                3315                3320

AGA AGT TTG AGC CCT GAG AAG ACA GCC TCA CTG ATC CAA GGA GGA ATC    10277
Arg Ser Leu Ser Pro Glu Lys Thr Ala Ser Leu Ile Gln Gly Gly Ile
              3325                3330                3335

GAT CGA CAG GAT GTC TCC GTG TAC GAC TCC CTC CTG ACT TCA GTT GGA    10325
Asp Arg Gln Asp Val Ser Val Tyr Asp Ser Leu Leu Thr Ser Val Gly
              3340                3345                3350

TTG GCG CTC CAA CAT ATC GCT CCA ACC GGC CCA AGT ATG GTT ACG ATC    10373
Leu Ala Leu Gln His Ile Ala Pro Thr Gly Pro Ser Met Val Thr Ile
3355                3360                3365                3370

GAG GGA CAT GGC CGT GAA GAA GTG GAT CAG ACA CTG GAT GTG AGC CGC    10421
Glu Gly His Gly Arg Glu Glu Val Asp Gln Thr Leu Asp Val Ser Arg
              3375                3380                3385

ACC ATG GGT TGG TTC ACC ACC ATG TAT CCA TTT GAA ATT CCC CGT CTC    10469
Thr Met Gly Trp Phe Thr Thr Met Tyr Pro Phe Glu Ile Pro Arg Leu
              3390                3395                3400

AGC ACC GAG AAC ATT GTT CAA GGA GTC GTC GCT GTG AGC GAA CGG TTC    10517
Ser Thr Glu Asn Ile Val Gln Gly Val Val Ala Val Ser Glu Arg Phe
              3405                3410                3415

AGA CAG GTG CCT GCC CGT GGC GTC GGG TAT GGA ACC TTG TAC GGC TAT    10565
Arg Gln Val Pro Ala Arg Gly Val Gly Tyr Gly Thr Leu Tyr Gly Tyr
              3420                3425                3430

ACT CAA CAC CCG CTG CCC CAG GTG ACC GTC AAC TAC CTG GGC CAG CTC    10613
Thr Gln His Pro Leu Pro Gln Val Thr Val Asn Tyr Leu Gly Gln Leu
3435                3440                3445                3450

GCC CGC AAG CAA TCG AAG CCA AAG GAA TGG GTC CTC GCG GTG GGC GAC    10661
Ala Arg Lys Gln Ser Lys Pro Lys Glu Trp Val Leu Ala Val Gly Asp
              3455                3460                3465

AAC GAA TTT GAA TAC GGA CTC ATG ACT AGC CCA GAG GAC AAA GAC CGG    10709
Asn Glu Phe Glu Tyr Gly Leu Met Thr Ser Pro Glu Asp Lys Asp Arg
              3470                3475                3480

AGC TCT TCT GCC GTC GAC GTC ACG GCC GTG TGT ATT GAC GGC ACT ATG    10757
Ser Ser Ser Ala Val Asp Val Thr Ala Val Cys Ile Asp Gly Thr Met
              3485                3490                3495

ATC ATC GAT GTG GAC AGT GCT TGG AGC CTT GAG GAG AGC GAG CAA TTC    10805
Ile Ile Asp Val Asp Ser Ala Trp Ser Leu Glu Glu Ser Glu Gln Phe
     3500                3505                3510
```

**Sequence Listing No. 1 (continued).**

```
ATC TCG AGC ATC GAG GAA GGA CTG AAC AAG ATC CTC GAC GGC AGG GCA        10853
Ile Ser Ser Ile Glu Glu Gly Leu Asn Lys Ile Leu Asp Gly Arg Ala
3515            3520            3525                  3530

AGT CAG CAA ACC TCG CGA TTC CCG GAT GTT CCT CAA CCG GCG GAG ACA        10901
Ser Gln Gln Thr Ser Arg Phe Pro Asp Val Pro Gln Pro Ala Glu Thr
            3535            3540                  3545

TAT ACG CCG TAT TTC GAG TAT CTG GAA CCT CCA CGA CAG GGA CCG ACG        10949
Tyr Thr Pro Tyr Phe Glu Tyr Leu Glu Pro Pro Arg Gln Gly Pro Thr
            3550            3555                  3560

CTG TTC CTG CTG CCG CCG GGC GAA GGA GGC GCC GAG AGT TAC TTC AAC        10997
Leu Phe Leu Leu Pro Pro Gly Glu Gly Gly Ala Glu Ser Tyr Phe Asn
        3565                3570            3575

AAC ATC GTC AAG CGC CTG CGT CAG ACA AAT ATG GTG GTC TTC AAC AAC        11045
Asn Ile Val Lys Arg Leu Arg Gln Thr Asn Met Val Val Phe Asn Asn
        3580                3585            3590

TAC TAC TTG CAC AGC AAA CGC CTG CGC ACG TTC GAG GAG CTG GCG GAA        11093
Tyr Tyr Leu His Ser Lys Arg Leu Arg Thr Phe Glu Glu Leu Ala Glu
3595                3600                3605            3610

ATG TAT CTC GAC CAA GTA CGC GGC ATC CAA CCA CAC GGA CCG TAC CAC        11141
Met Tyr Leu Asp Gln Val Arg Gly Ile Gln Pro His Gly Pro Tyr His
            3615                3620                  3625

TTC ATC GGA TGG AGC TTC GGA GGA ATT CTC GCA ATG GAA ATG TCG CGG        11189
Phe Ile Gly Trp Ser Phe Gly Gly Ile Leu Ala Met Glu Met Ser Arg
            3630                3635                  3640

CGA CTG GTA GCC TCG GAC GAG AAG ATT GGC TTC CTC GGT ATT ATC GAC        11237
Arg Leu Val Ala Ser Asp Glu Lys Ile Gly Phe Leu Gly Ile Ile Asp
        3645                3650                  3655

ACC TAT TTC AAC GTG CGG GGA GCG ACA CGC ACC ATT GGC TTG GGG GAC        11285
Thr Tyr Phe Asn Val Arg Gly Ala Thr Arg Thr Ile Gly Leu Gly Asp
        3660                3665                  3670

ACT GAG ATT CTG GAC CCG ATC CAT CAC ATC TAC AAT CCC GAT CCG GCC        11333
Thr Glu Ile Leu Asp Pro Ile His His Ile Tyr Asn Pro Asp Pro Ala
3675                3680                3685                  3690

AAC TTC CAA CGC CTG CCC TCT GCA ACA GAT CGC ATT GTG CTG TTC AAG        11381
Asn Phe Gln Arg Leu Pro Ser Ala Thr Asp Arg Ile Val Leu Phe Lys
            3695                3700                  3705

GCC ATG AGG CCG AAC AAC AAG TAC GAA TCC GAG AAC CAG CGT CGC CTG        11429
Ala Met Arg Pro Asn Asn Lys Tyr Glu Ser Glu Asn Gln Arg Arg Leu
            3710                3715                  3720

TAC GAG TAC TAT GAC CGC ACT CGA CTC AAC GGA CTG GAC AGC TTG TTA        11477
Tyr Glu Tyr Tyr Asp Gly Thr Arg Leu Asn Gly Leu Asp Ser Leu Leu
            3725                3730                  3735
```

35

Sequence Listing No. 1 (continued)

```
CCA AGC GAT TCC GAC GTC CAG CTG GTC CCG CTT ACG GAC GAT ACA CAC        11525
Pro Ser Asp Ser Asp Val Gln Leu Val Pro Leu Thr Asp Asp Thr His
    3740              3745              3750

TTT TCC TGG GTC GGA AAT CCA CAA CAG GTG GAG CAG ATG TGT TCG ACT        11573
Phe Ser Trp Val Gly Asn Pro Gln Gln Val Glu Gln Met Cys Ala Thr
3755              3760              3765              3770

ATC AAG GAA CAC CTC GCT CGC TAT TGA                                    11600
Ile Lys Glu His Leu Ala Arg Tyr End
        3775


TCCGTCACTA GCAGCACAGT ATATCGGACG ATGGAAGTGA TGGAGTGGGG GGGATAGGAT      11660
ACGATCAAAC CAGGGTGCGG TTCTTTTTGG GGGGAACTAG TCTCTGGTTG AGGAAAGCGA      11720
GGTAGCAAAT AAACTACCAA GGTCTAGACC CACATAGTCT GTCATTGTTT TCGATCCTAA      11780
ATTGATATAT AATAGGCGAC ACCTTTAGTT AGCCAAATTT TCTATATAGA AACACCACGG      11840
TTTTTAGGAG TTAGGACCAC GTCAGACCGT GGCCCTTTCA CTAACGCTTC CGTTTACATC      11900
CATACCGGAT GTGGTTGTAG CACATTTATA TGATATCATT TAAGACTATA TACGCCTATT      11960
CCCCCCTATC GAATAGGCCC CTACGTATTT CTTTTGTTTT TTTCTTTTTC TTTTTTTTTT      12020
TTTTCTTTTC GCTCTCTCCC CTTTTATACC CAATATCGGA TCGAGTTGAT AATATCAATA      12080
TCTAAAACTC CCAATTAAAC CTACAAAGCC TATCTTAGTG TAAGTGAATT TGGGCTCTGG      12140
ACCAAATTCT CCGCCAAGGA TAATCTTTCC GATAAACGGT GGTTATCCGG TCATCATAAA      12200
AAAGGAAAAG GTACTCCGTC CTCGATAATA AAACGTAACA TAAGCATGTC GTTCACCATA      12260
GACAAGAGGA ACCAACATCA TTAAGCAGGG GATAGGTTCA TCCGGTCTAG GGCGTCGAGT      12320
GCCACCGCCC GTAGGTTGTC AATCTTGAGC TGGATTTGGA GATC                       12364
```

## Claims

1. A mutant δ-(L-α-aminoadipyl)-L-cysteinyl-D-valine synthetase (ACVS') comprising at least a functional portion of ACVS and characterized as differing in at least on amino acid from a wild-type ACVS.

2. ACVS' according to Claim 1, wherein said mutant is characterized as having an amended substrate specificity as compared to wild-type ACVS.

3. ACVS' according to Claim 1 or 2 characterized as having increased efficiency in the production of tripeptides as compared to wild-type ACVS.

4. A method for the preparation of ACVS' according to any of the preceding claims, said method comprising: altering a DNA sequence encoding wild-type ACVS, preferably obtainable from the gene encoding said Penicillium chrysogenum or Escherichia coli, by mutagenesis.

5. A method according to Claim 4, wherein said in vitro mutagenesis is of any of domains I, II, III or IV as indicated in Figure 15, or by exchange thereof.

6. A method according to Claim 4, wherein said in vitro mutagenesis is of any of the subdomains as indicated in Figure 15.

7. A DNA sequence encoding ACVS' according to Claims 1 or 2.

8. A DNA construct comprising a DNA sequence encoding ACVS' according to Claims 1 or 2.

9. A DNA construct according to Claim 8, adapted for expression of the said DNA sequence in filamentous fungi, preferably in Penicillium chrysogenum or Acremonium chrysogenum.

10. A DNA construct according to Claim 9, adapted for expression preferably in the vector pPCV01.

**11.** A DNA construct according to Claim 8, adapted for expression of the said DNA sequence in a prokaryotic host.

**12.** A DNA construct selected from the group consisting of pMA-ACVS, pSLACV-01, pSLACV-03A or pSLACV-03B.

**13.** A transformed host cell comprising a DNA construct according to any one of the Claims 8-12, preferably selected from the group consisting of: Escherichia coli, Streptomycetes or filamentous fungi, preferably Penicillium chrysogenum or Acremonium chrysogenum.

**14.** A method for obtaining or enhancing the production of a fermentable or known or new β-lactam antibiotic in a host microbial cell comprising:
transforming a host cell with a DNA construct according to any one of the Claims 8, 9 or 10;
cloning the resulting transformants; and
identifying clones of transformants having enhanced production of said antibiotic in comparison to an untransformed host cell.

**15.** A method according to Claim 14, wherein said host cell is a filamentous fungus, preferably Penicillium chrysogenum or Acremonium chrysogenum, or a Streptomyces.

**16.** A method for the preparation of a mutant δ-(L-α-aminoadipyl)-L-cysteinyl-D-valine synthetase enzyme using a transformant according to Claim 11 or 12 and isolating the enzyme formed.

**17.** A method for in vitro synthesis of ACVS' according to any one of the Claims 1, 2 or 3 wherein said synthesis is performed using the enzyme prepared according to Claim 14.

## DISTRIBUTION OF ß-LACTAM PRODUCING MICROORGANISMS

| Class of ß-lactams | Fungi | Bacteria | |
|---|---|---|---|
| | | Gram positive | Gram negative |
| PENAM | Aspergillus Penicillium Epidemophyton Trichophyton Polypaecillum Malbranchea Pleurophomopsis | | |
| CEPHEM | Cephalosporium Spiroidium Scopulariopsis Diheterospora Peacilomyces | Streptomyces Nocardia | Flavobacterium Xanthomonas Lysobacter |
| CLAVAM | | Streptomyces | |
| CARBAPENEM | | Streptomyces | Serratia Erwinia |
| MONOBACTAM | | Nocardia | Pseudomonas Gluconobacter Chromobacterium Agrobacterium Acetobacter |

**FIGURE 1**

**FIGURE 2**

FIGURE 3

FIGURE 4A

(Rsa I)  Pvu II  Pvu II  Nhe I  Xmn I  Pvu II  Xmn I  Sac II  BamHI  Acc I  Ava I  EcoRV  Pvu II  Bst EII  (Rsa I)  Ava I  Sal I Acc I HincII

E

8250  8500  8750  9000  9250  9500  9750  10000  10190

Sal I Acc I HincII  (Rsa I)  Xmn I  Dra I  Xba I  Ava I  Sac I  Sac I  (Rsa I)  Pvu II  Sac II  EcoRV  EcoRI  BamHI  Ava I  EcoRV  HincII  HincII  Xho I  Xho I  Nco I  Ava I  Ava I  Sac I  EcoRI  EcoRI HincII  Sal I Acc I HincII

E  F  G  H

GTA

10185  10250  10500  10750  11000  11250  11500  11750  12000  12250  12363

Sal I Acc I HincII  Apa I  Ava I  Sac I  Pst I  Xba I  Nco I  HincII  HincII  Pst I  Bst EII  EcoRV  BamHI

12358  12500  12750  13000  13250  13500  13750  14000

FIGURE 4B

42

**FIGURE 5**

**FIGURE 6**

**FIGURE 7**

FIGURE 8

46

**FIGURE 9**

XbaI SpeI

AmpR

F1or ori

Fdter

14000

2000

EcoRI

NdeI

ptrp

12000

pMA-ACVS

15 kbp

4000

10000

8000

6000

pcbAB

XbaI

**FIGURE 10**

**FIGURE 11**

**FIGURE 12A**

ScaI

BglII
SstI

AflII

KpnI

EcoRV
ClaI

StuI

II
18000

mel

16000

2000

tsr

14000    pSLACV-03B    4000

Apa I

18    bp

12000    6000

pc ACVS

XhoI
SpeI
ScaI

10000    8000

BglII    BglI    DraI    XbaI

**FIGURE 12B**

**FIGURE 13A**

```
P. chrysogenum ACV Synthetase A( 301-1068)   -----SAEQKQ-QLEEWNNTDGEYPSSKRLHHLIEEVVERHEDKIAVVCDERELTY   350
P. chrysogenum ACV Synthetase B(1392-2154)   -----SSIQLE-QLAAWNATEAFFPDTT-LHEMFENEASQKPDKIAVVYEETSLTY   1445
P. chrysogenum ACV Synthetase C(2474-3295)   RPTENGDIHLPLAQSPLATTAEEQKVAS-LHQAFEREAFLAAEKIAVVQGDRALSY   2528
B. brevis Tyrocidine Synthetase I   (1-798)  MLANQANLIDNKRELEQHALVPYAQGKS-IHQLFEEQAEAFPDRVAIVFENRRLSY   55
B. brevis Gramicidine Synthetase I(13-809)   QNKNGTHEEEQYLFAVNNTKAEYPRDKT-IHQLFEEQVSKRPNNVAIVCENEQLTY   67


GELNAQGNSLARYLRS-IGILPEQLVALFLDKSEKLIVTIIGVWKSGAAVVPIDPTYVPDERVRFVLDDTKARAIIASNQHVERLQREVIGDRNLCIIRLE   449
RELNERAHRMAHQLRSDVSPNPNEVIALVMDKSEHMIVNILAVWKSGGAYVPIDPGYPNDRIQYILEDTQALAVIADSCYLPRI-KGMAASGTL----LY   1540
ADLNGQANQLARYIQSVSCIGADDGIALHLEKSIDTIICILAIWKAGAAVVPLDPTYPPGRVQLILEEIKAKAVLVHSSHASKC-ERHGAKVIA----VD   2623
QELNRKANQLARALLEKG-VQTDSIVGVMMEKSTENVIAILAVLKAGGAYVPIDIEYPRDRIQYILQDSQTKIVLTQKS-VSQ--LVHDVGYSG----EV   147
HELNVKANQLARIFIEKG-IGKDTLVGIMMEKSTDIFIGILAVLKAGGAYVPIDIEYPKERIQYILDDSQARMLLTQKH-LVH--LIHNIQFNG----QV   159


PLIASLAQDSSKFPAHNLDDLPLTSQQLAYVTYTSGTTGFPKGIFKQHTNVVNSITDLSARYGVAG-QHHEA-ILLFSACVFEPFVRQTLMALVNGHLLA   547
PSVLPAHPDS-KWSVSNPSPLS-RSTDLAYIIYTSGTTGRPKGVTVEHHGVVNLQVSLSKVFGLRD-TDDEV-IISFSNYVFDHFVEQMTDAILNGQTLL   1636
SPAIETAVSQ-Q-SAADLPTIA-SLGNLAYIIFTSGTSGKPKGVLVEQKAVLLLRDALRERYFGRDCTKHHG-VLFLSNYVFDFSVEQLVLSVLSGHKLI   2719
VVLDEEQLDA--RETANLHQPS-KPTDLAYVIYTSGTTGKPKGTMLEHKGI-AICNPFSKIRLASPSKTGSG-FLPACRSTHPFGKCSW-LCCL-APRVH   240
EIFEEDTIKI--REGTNLHVPS-KSTDLAYVIYTSGTTGNPKGTMLEHKGI-SNLKVFFENSLNVTEKDRIGQFASISFDASVWEMFMA-LLTG-ASLYI   253


VINDVEKYDADTLLPFIRRHSITYLNGTASVLQEVDFSDCP-SLNRIILVGENITEARYLALRQRFKNRILNEYGFTESAFVTALKIFDPESTRKDTSLG   646
VLNDGMRGDKERLYRYIEKNRVTYLSGTPSVVSMYEFSRFKDHLRRVDCVGEAFSEPVFDKIRETFHGLVINGYGPTEVSITTHKRLYPFPERRMDKSIG   1736
VPPAEFVADDE-FYRMASTHGLSYLSGTPSLLQKIDLARL-DHLQVVTAAGEEIHATQYEKMRRRFNGPIYNAYGVTETTVYNIIAEFT-TNSTFENALR   2816
PSKQTIHDFAA-FEHYLSENELTIITLPPTYLTHLTPERI-TSLRIMITAGSASSAPLVNKWKDKL--RYINAYGPTETSICATIWEAP-SNQLSVQSVP   335
ILKDTINDFVK-FEQYINQKEITVITLPPTIVVHLDPERI-LSIQTLITAGSATSPSLVNKWKEKV--TYINAYGPTETTICATTWVAT-KETIG-HSVP   347


--RPVRNVKCYILNPSLKRVPIGATGELHIGGLGISKGYLNRPELTPHREIPNPFQTDCEKQLGINSLMYKTGDLAR--WLP--NGEVEYLGRADFQIKL   740
--QQVHNSTSYVLNEDMKRTFIGAVGELYLGGEGVVRGYHNRADVTAERFIPNPFQSEEDKREGRNSRLYKTGDLVR--WIPGSSGEVEYLGRNDFQVKI   1832
--EVLPGTRAIVINAALQPVPFDAVGELYLAGDSVTRGYINQPLLTDQRFIPNPFCKEEDIAMGRFARLYKTGDLVRSRFNRQQQPQLEYLGRGDLQIKM   2914
IGKPIQNTHIYIVNEDLQLLPTADEGELCIGGVGLARGYWNRPDLTAEKEVDNPF--------VPGEKMYRTGDL----AKWLTDGTIEFLGRIDHQVKI   423
IGAPIQNTQIYIVDENLQLKSVGEAGELCIGGEGLARGYWKRPELTSQKEVDNPF--------VPGEKLYKTGDQ----ARWLSDGNIEYLGRIDNQVKI   435


RGIRIEPGEIETMLAMYPRVRTSLVVSKKLRNGPEETTNEHLVGYYVCDSASVSEADLLSFLEKKLPRYMIPTRLV-QLSQIPVNVNGKADLRALPAV--   837
RGLRIELGEIEAILSSYHGIKQSVVIAKDCREG-----AQKFLVGYYVADAA-LPSAAIRRFMQSRLPGYMVPSRLI-LVSKFPVTPSGKLDTKALPPA--   1924
RGYRIEISEVQNVLTSSPGVREGAVVAKYENHDTYSRTAHSLVGYVTTDNETVSEADILTFMKARLPTYMVPSHLCCLEGALPVTINGKLDVRRLPE--I   3012
RGHRIELGELESVLLAHEHITEAVVIAR-----EDQHAGQYLCAYVISQQEATP-AQLRDYAAQKLPAYMLPSYFVKLD-KMPLTPNDKIDRKALPEPDL   516
RGHRVELEEVESILLKHMYISETAVSVH-----KDHQEQPYLCAYFVSEKHIPL-EQLRQFSSEELPTYMIPSYFIQLD-KMPLTSNGKIDRKQLPEPDL   528
```

53

FIGURE 13B

```
-DISNSTEVRSDLRGDTEIALGEIWADVLGARQRSVSRNDNFFRLGGHSITCIQLIARIRQRQRLSVSISVEDVFATRTLERMADLLQNKQQEKCD-KPH   935
-EEESEIDVVPP-RSEIERSLCDIWAELIEMHPEETGIVSDFFSIGDDSLKSTKL--SFMTHESFNRAVSVSALECHRTVEAQTHLILHDAADVHEIMPI   2020
INDSAQSSYSPP-RNIIEAKMCRLWESALGM--FRCGTDDDLFKIGGDSITSLHL--VAQTHNQVGCKITVRDIFEHRTARALHDHVEMKDSDRSNULQE   3107
TANQSQAAYHPP-RTETESILVSIWQNVLGI--EKIGIRDNFYSIGGDSIQAIQV--VARLHS-YQLKLETKDLLNYPTIEQV--ALFVESTTRKSDQGI   608
TFGM-RVDYEAP-RNEIEETLVTIWQDVLGI--EKIGIKDNFYAIGGDSIKAIQV--AARLHS-YQLKLETKDLLKYPTIDQL--VHYIKDSKRRSEQGI   619

E-APTELL---EENAATDNIYLANSLQQGFVYHYLKSMEQSDAYVMQSVLRYNTTLSPDLFQRAWKHAQQSFPALR--LRFSWEKEVFQLLDQDP-PLDW   1028
DCNDTQMI---PVSRAQERLLFIHEFENGSNAYNIDAAFELPGSVDASLLEQALRGNLARHEALRTLLVKDHATGI--YLQKVLSPDEAQGMFSV-NVDT   2114
RTEQGPVIGEAPLLPIQDWFL-SKALQH--PMYWNHTFYVRTPELDVDSLSAAVRDLQQYHDVFRMRLKREEVGFVQ-SFAEDFSPAQLRVLNVK-DVDG   3202
IAGNVPLTPIQKWF-----FG-KNFTNT--GHWNQSSVLYRPEGFDPKVIQSVMDKIIEHHDAVRMVYQHENGNVVQHNRGLGGQLYDFFSYNLTAQPDV   700
VEGEIGLTPIQHWF-----FE-QQFTNM--HHWNQSYMLYRPNGFDKEILLRVFNKIVEHHDALRMIYKHHNGKIVQINRGLEGTLFDFYTFDLTANDNE   711

RFLYFTDVAAGAVEDRKLEDLRRQDLTERFKLDVGRLFRV                                                               1068
AKQVERLDQEIASLSQHVFRLDDELPWEARILKLESGGLY                                                               2154
SAAVNEILDGWQSGFNLENGPIGSIGYLHGYEDRSARVWFSVHHMAIDTVSWQILVRDLQTLYRNGSLGSKGSSFRQWA---EAIQNYKASDSERN----  3295
QQAIEAETQRLHSSMNLQEGPLVKVALFQTL--HGDHFFLAIHHLVVDGISWRILFKIWQPDTRRHLQGKRSVCPKKRILFKAGHNGCKINAHEADLLSE   798
QQVICEESARLQNSINLEVGPLVKIALFHTQ--NGDHLFMAIHHLVVDGISWRILFEDLATAYEQAMHQQTIALPEKTDSFKDWSIELEKYANSELFLEE   809
```

Pc ACVS P                                                                LIMITS:  3563  364
ratfas

```
3563            L  F  L  L  P  P  G  E  G  G  A  E  S  Y  F  N  N
                !  !  !  :     !     !  !                 !
1         E  R  P  L  F  L  V  H  P  I  E  G  S. I  T  V  H  F  H  S

3580      I  V  K  R  L  R  Q  T  N  M  V  V  F  N  N  Y  Y  L  H  S
          :           :  !                 :     :                 :
21        L  A  A  K  L  S  V  P  T  Y  G  L     Q  C        T  Q  A

3600      K  R  L  R  T  F  E  E  L  A  E  M  Y  L  D  Q  V  R  G  I
             !        :        !  !        !  :  !     :  :     :
38        A  P  L  D  S  I  P  N  L  A  A  Y  Y  I  D  C  I  K  Q  V

3620      Q  P  H  G  P  Y  H  F  I  G  W  S  F  G  G  I  L  A  M  E
          !  !     !  !  !  :        !  :  !  !  !        :  !     !
58        Q  P  E  G  P  Y  R  V  A  G  Y  S  F  G  A  C  V  A  F  E

3640      M  S  R  R  L  V  A  S
          !           !     !
78        M  C  S  Q  L  Q  A  Q  Q
```

Matches = 27      Mismatches = 55      Unmatched = 7
Length = 89       Matches/length =  30.3 percent

**FIGURE 14**

DOMAINS AND SUBDOMAINS WITHIN THE ACVS PROTEIN

| Domain | position (amino acid) | function | subdomains |
|--------|----------------------|----------|------------|
| I. | 301-1068 | activation of amino acid substrate | 374- 423<br>474- 501<br>655- 699<br>725- 754 |
| II. | 1392-2154 | idem | 1470-1518<br>1564-1590<br>1745-1789<br>1817-1846 |
| III. | 2474-3295 | idem | 2554-2603<br>2647-2673<br>2827-2871<br>2899-2928 |
| IV. | 3560-3647 | thioesterase | |

FIGURE 15

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 320 272  (BEECHAM GROUP LTD)<br>* Claims; page 8, line 56 - page 9, line 6 *<br>– – – | 1-4,7-9,<br>13-17 | C 12 N 9/00<br>C 12 N 15/52<br>C 12 N 1/21 |
| D,Y | THE JOURNAL OF BIOLOGICAL CHEMISTRY, vol. 264, no. 7, March 1989, pages 3680-3684; H. VAN LIEMPT et al.: "Gamma-(L-alpha-aminoadipyl)-L-cysteinyl-D-valine synthetase from Aspergillus nidulans"<br>* Whole article *<br>– – – | 1-17 | C 12 N 1/15<br>C 12 P 35/00<br>C 12 P 37/00 |
| P,Y | THE JOURNAL OF BIOLOGICAL CHEMISTRY, vol. 265, no. 27, September 1990, pages 16358-16365; B. DIEZ et al.: "The cluster of penicillin biosynthetic genes"<br>* Whole article *<br>– – – | 1-17 | |
| D,P,Y | EP-A-0 357 119  (GIST-BROCADES)<br>* Claims; page 9, lines 4-9 *<br>– – – – – | 1-17 | |

|  |
|---|
| **TECHNICAL FIELDS SEARCHED (Int. Cl.5)** |
| C 12 N |

The present search report has been drawn up for all claims

| Place of search | Date of completion of search | Examiner |
|---|---|---|
| The Hague | 03 June 91 | HUBER A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document